# EUROPEAN PATENT APPLICATION

(11) **EP 2 666 766 A1**
(43) Date of publication of application: **27.11.2013**
(21) Application number: 12168638.0
(22) Date of filing: 21.05.2012
(51) Int. Cl.: C07C 271/16, C07C 275/24, C07D 213/30, C07D 261/18, C07D 263/58, C07D 317/58, A61P 17/00, A61K 31/42, A61K 31/17, A61K 31/16, A61Q 19/00

(54) **Novel E,E-diene compounds and their use as medicaments and cosmetics**

(71) Applicant: Dr. August Wolff GmbH & Co. KG Arzneimittel, 33611 Bielefeld (DE)
(72) Inventor: Soeberdt, Michael, 33605 Bielefeld (DE); Knie, Ulrich, 32105 Bad Salzuflen (DE); Abels, Christoph, 33619 Bielefeld (DE)
(74) Representative: Ter Meer Steinmeister & Partner

(57) **Abstract**

The present invention relates to a novel class of *E,E-*diene compounds and their use as a medicament, preferably as a dermatologic agent, and as a cosmetic. These novel compounds are particularly useful in treating and/or preventing inflammation, irritation, itching, pruritus, pain, oedema and/or pro-allergic or allergic conditions in a patient. Usually they are topically applied to the skin or mucosa in the form of a pharmaceutical or cosmetic composition comprising the compound and a pharmaceutically and/or cosmetically acceptable carrier.

## Description

The present invention relates to a novel class of *E,E*-diene compounds and their use as a medicament, preferably as a dermatologic agent, and as cosmetics. These novel compounds target the endocannabinoid system and are particularly useful in treating and/or preventing inflammation, irritation, itching, pruritus, pain, oedema, and/or pro-allergic or allergic conditions in a patient. Usually they are topically applied to the skin or mucosa of a mammal in the form of a pharmaceutical or cosmetic composition comprising the compound and a pharmaceutically and/or cosmetically acceptable carrier.

### Background of the Invention

The endocannabinoid system (ECS) comprises cannabinoid receptors (CB₁ and CB₂, TRPV1 and potentially also GPR55), arachidonic acid-derived ligands, and their regulatory enzymes. The importance of the endocannabinoid system in peripheral tissues has been demonstrated in numerous recent studies (Di Marzo V. Targetin the endocannabinoid system: to enhance or reduce? Nat Rev Drug Discov. 2008 May; 7(5):438-55). Whereas the activation of the peripheral endocannabinoid system is often associated with anti-inflammatory and immunosuppressive effects, in the skin the role of the ECS is more complex. CB₂ receptor activation has been shown to trigger endorphins that locally act analgesic (Ibrahim MM, Porreca F, Lai J, Albrecht PJ, Rice FL, Khodorova A, Davar G, Makriyannis A, Vanderah TW, Mata HP, Malan TP Jr. CB2 cannabinoid receptor activation produces antinociception by stimulating_ peripheral release of endogenous opioids. Proc Natl Acad Sci U S A. 2005 Feb 22;102(8):3093-8). Studying CB knockout mice, it has been observed that the ECS and the CB₁ receptor can inhibit the pathogenesis of allergic contact dermatitis (Karsak M, Gaffal E, Date R, Wang-Eckhardt L, Rehnelt J, Petrosino S, Starowicz K, Steuder R, Schlicker E, Cravatt B, Mechoulam R, Buettner R, Werner S, Di Marzo V, Tüting T, Zimmer A. Attenuation of allergic contact dermatitis through the endocannabinoid system. Science. 2007 Jun 8;316(5830):1494-7). Interestingly, in this study it was shown that inhibitors of anandamide degradation via fatty acid amide hydrolase (FAAH) could be a promising therapeutic strategy to treat different forms of dermatitis. Overall, the endocannabinoid system in the skin is present, both CB₁ and CB₂ receptors have been detected in keratinocytes and fibroblasts and the endocannabinoids are released in the skin where they seem to regulate multiple signals involved in inflammation. Anandamide exerts potent anti-inflammatory and anti-allergic effects (Leonti M, Casu L, Raduner S, Cottiglia F, Floris C, Altmann KH, Gertsch J. Falcarinol is a covalent cannabinoid CB1 receptor antagonists and induces pro-allergic effects in skin. Biochem. Pharmacol. 2010, 79: 1815-1826). Moreover, anandamide reuptake inhibitors have been shown to exert a number of beneficial effects including neuropathic and peripheral pain (Yates M L, Baker EL. Inactivation and Biotransformation of the endogenous cannabinoids anadamide and 2-arachidonoyl glycerol. Mol. Pharmacol. 2009, 76: 11-17). However, the mechanisms underlying are not yet fully understood and there still remain numerous questions to be answered.

JP2003238567 discloses an antitumor benzolactone enamide which is manufactured by fermentation from Pseudomonas. The enamide function contains a 6-oxa-hepta-*2E,4E*-dienoyl or 6-oxa-hepta-*2Z,4Z*-dienoyl residue.

Aumann et al. describe organic syntheses via transition metal complexes (Eur. J. Org. Chem. 2000, 1, 37-49).

Fischer and Neuenschwander describe rearrangements of aminopentadienals (Helv. Chim. Acta 1998, 81,2282-2291.

Blaskovich and Kahn have used the Stille reaction for the preparation of functionalized dienes on solid support (J. Org. Chem. 1998, 63, 1119-1125).

Cosset et al. describe the synthesis and reactivity of new Fischer type enynylcarbene tungsten complexes via 1,4-diyn-3-ols (Synlett 1996, 5, 435-436).

Gutierez et al. describe the intramolecular Diels-Alder cycloaddition of N-dienoyl acrylimidates (J. Org. Chem. 1989, 54,4335-4344).

Gras et al. describe the synthesis of alpha-functionalized allenes (Bull. Soc. Chim. Fr. 1988, 4, 757-767).

Dornow and Ische describe the preparation of Mannich bases from oxoacetylene compounds (Chem. Ber. 1956, 89, 870-876).

WO2010/136221 discloses dodeca-*2E,4E*-diene amides and their use as medicaments and cosmetics.

Thus, the claimed class of *E,E*-diene compounds of the present invention is not disclosed in the prior art, let alone to be suitable and active in the treatment and prevention of skin disorders.

Further, even if a large number of dermatologic agents and preparations is known in the prior art for treating any kinds of skin discomfort, there is a still a strong demand to find new active agents being more effective, requiring even reduced amounts to be applied, and having less undesirable side-effects.

Thus, the object underlying the present invention is the provision of new compounds suitable as medicaments. It is a further object of the present invention to provide for new compounds suitable as dermatologic agents for treating and preventing several conditions including inflammation, irritation, itching, pruritus, pain, oedema, and/or pro-allergic or allergic conditions etc., particularly of the skin and mucosa of a mammal, which compounds are more effective, require reduced amounts of active ingredient as compared to prior art compounds and, and have less undesirable side-effects.

### Summary of the Invention

The objects as mentioned above have surprisingly been solved in accordance with the present invention. Thus, the present invention relates to specific *E*,*E*-diene compounds according to formula (1) below.

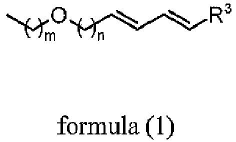

The compounds according to formula (1) are alternative solutions to the same problem and have not been obvious for the skilled person.

In formula (1) the residue R³ is selected from the group consisting of -CH₂N(R⁴)C(O)R¹, - CH₂N(R⁴)C(O)NR¹R², -CH₂N(R⁴)C(O)OR¹ and -C(O)NR¹R².

In formula (1) n is defined as 1, 2, 3, 4, or 5; m is defined as 0, 1, 2, 3, or 4; and m + n is defined as 1, 2, 3, 4, 5, 6 or 7.

In the four above mentioned respective definitions of R³ the residues R¹ and R² are independently selected from hydrogen, C₁₋₆ alkyl, C₃₋₇ cycloalkyl, C₆₋₁₀ aryl, 5- to 10-membered heteroaryl, (C₆₋₁₀ aryl)-C₁₋₆ alkyl, (5- to 10-membered heteroaryl)-C₁₋₆ alkyl, (C₆₋₁₀ aryl fused with a 4 to 8-membered saturated or unsaturated heterocyclyl containing 1 to 3 heteroatoms independently selected from N, O, S), (C₆₋₁₀ aryl fused with a 4 to 8-membered saturated or unsaturated heterocyclyl containing 1 to 3 heteroatoms independently selected from N, O, S)-C₁₋₆alkyl, C₁₋₆alkyl-C(O)OC₁₋₆alkyl, C₁₋₆alkyl-C(O)NH(C₁₋₆alkyl), C₁₋₆alkyl-C(O)N(C₁₋₆alkyl)₂, C₁₋₆alkyl-CN.

Moreover, R⁴ is hydrogen or C₁₋₆-alkyl.

Optionally, in formula (1) R¹, R², and R⁴, independently of each other, are substituted by one, two or three substituents R⁵, wherein R⁵ is selected from the group consisting of halogen; CN; OH; oxo; C₁₋₆-alkyl, optionally substituted with one or more substituents selected from halogen, OH, OC₁₋₆alkyl, OC₃₋₇cycloalkyl; OC₁₋₆-alkyl, optionally substituted with one or more substituents selected from halogen, OH, OC₁₋₆alkyl, OC₃₋₇cycloalkyl; C₆₋₁₀-aryl; 5- to 10-membered heteroaryl; 5- to 11-membered heterocyclyl, optionally substituted with one or more substituents selected from halogen, OH, oxo, OC₁₋₆alkyl, OC₃₋₇cycloalkyl; C(O)NH₂; C(O)OH; C(O)OC₁₋₆alkyl, optionally substituted with one or more substituents selected from halogen, OH, OC₁₋₆alkyl, OC₃₋₇cycloalkyl; C(O)OC₃₋₇cycloalkyl, optionally substituted with one or more substituents selected from halogen, OH, OC₁₋₆alkyl, OC₃₋₇cycloalkyl; C(O)NH-C₁₋₆alkyl, optionally substituted with one or more substituents selected from halogen, OH, OC₁₋₆alkyl, OC₃₋₇cycloalkyl; C(O)NH-C₃₋₇cycloalkyl, optionally substituted with one or more substituents selected from halogen, OH, oxo, OC₁₋₆alkyl, OC₃₋₇cycloalkyl; C(O)N-(C₁₋₆ alkyl)₂, optionally substituted with one or more substituents selected from halogen, OH, OC₁₋₆ alkyl, OC₃₋₇cycloalkyl; C(O)N-(C₁₋₆alkyl)(C₃₋₇cycloalkyl), optionally substituted with one or more substituents selected from halogen, OH, OC₁₋₆alkyl, OC₃₋₇cycloalkyl; SO₂-C₁₋₆alkyl, optionally substituted with one or more substituents selected from halogen, OH, OC₁₋₆alkyl, OC₃₋₇cycloalkyl; NH₂; NH-C₁₋₆alkyl, optionally substituted with one or more substituents selected from halogen, OH, OC₁₋₆alkyl, OC₃₋₇cycloalkyl; N-(C₁₋₆alkyl)₂, optionally substituted with one or more substituents selected from halogen, OH, OC₁₋₆alkyl, OC₃₋₇cycloalkyl; NH-SO₂-CH₃; NH-C(O)OC₁₋₆alkyl, optionally substituted with one or more substituents selected from halogen, OH, OC₁₋₆alkyl, OC₃₋₇cycloalkyl; NH-C(O)OC₃₋₇cycloalkyl, optionally substituted with one or more substituents selected from halogen, OH, OC₁₋₆alkyl, OC₃₋₇ cycloalkyl; N(C₁₋₆alkyl)-C(O)OC₁₋₆alkyl, optionally substituted with one or more substituents selected from halogen, OH, OC₁₋₆alkyl, OC₃₋₇cycloalkyl; N(C₁₋₆alkyl)-C(O)OC₃₋₇ cycloalkyl, optionally substituted with one or more substituents selected from halogen, OH, OC₁₋₆alkyl, OC₃₋₇cycloalkyl; NH-C(O)C₁₋₆alkyl, optionally substituted with one or more substituents selected from halogen, OH, OC₁₋₆alkyl, OC₃₋₇cycloalkyl; NH-C(O)C₃₋₇ cycloalkyl, optionally substituted with one or more substituents selected from halogen, OH, OC₁₋₆alkyl, OC₃₋₇cycloalkyl; N(C₁₋₆alkyl)-C(O)C₁₋₆alkyl, optionally substituted with one or more substituents selected from halogen, OH, OC₁₋₆alkyl, OC₃₋₇cycloalkyl; and N(C₁₋₆alkyl)-C(O)C₃₋₇cycloalkyl, optionally substituted with one or more substituents selected from halogen, OH, OC₁₋₆alkyl, OC₃₋₇cycloalkyl.

Preferably in the compounds of formula (1) n is 1 or 2, more preferably 2; m is 3 or 4, more preferably 3; and R³ is selected from the group consisting of -CH₂N(R⁴)C(O)OR¹and - CH₂N(R⁴)C(O)NR¹R² with R¹ and R² and R⁴ as defined above.

More preferably in the compounds of formula (1), in the NR¹R² moieties, one of R¹ and R² is hydrogen and the other is selected from (C₆₋₁₀ aryl)-C₁₋₆ alkyl, (5- to 10-membered heteroaryl)-C₁₋₆ alkyl, (C₆₋₁₀ aryl fused with a 4 to 8-membered saturated or unsaturated heterocyclyl containing 1 to 3 heteroatoms independently selected from N, O, S), (C₆₋₁₀ aryl fused with a 4 to 8-membered saturated or unsaturated heterocyclyl containing 1 to 3 heteroatoms independently selected from N, O, S)-C₁₋₆alkyl;
and in the C(O)R¹ and C(O)OR¹ moieties, R¹ is selected from C₆₋₁₀ aryl, 5- to 10-membered heteroaryl, (C₆₋₁₀ aryl)-C₁₋₆ alkyl, (5- to 10-membered hoteroaryl)-C₁₋₆ alkyl, (C₆₋₁₀ aryl fused with a 4 to 8-membered saturated or unsaturated heterocyclyl containing 1 to 3 heteroatoms independently selected from N, O, S), (C₆₋₁₀ aryl fused with a 4 to 8-membered saturated or unsaturated heterocyclyl containing 1 to 3 heteroatoms independently selected from N, O, S)-C₁₋₆alkyl, C₁₋₆alkyl-C(O)OC₁₋₆alkyl, C₁₋₆alkyl-C(O)NH(C₁₋₆alkyl), C₁₋₆alkyl-C(O)N(C₁₋₆ alkyl)₂, and C₁₋₆alkyl-CN.

Even more preferably in the compounds of formula (1) R³ is -CH₂N(R⁴)C(O)OR¹, R¹ is selected from C₆₋₁₀ aryl, (C₆₋₁₀ aryl)-C₁₋₆ alkyl, C₁₋₆alkyl-CN, C₁₋₆alkyl-C(O)NH(C₁₋₆alkyl), C₁₋₆alkyl-C(O)N(C₁₋₆alkyl)₂, and (5- to 10-membered heteroaryl)-C₁₋₆ alkyl; and R⁴ is either hydrogen or methyl.

In the above mentioned definitions of formula (1) R¹ and R², independently of each other, preferably are substituted by one or two substituents R⁵, more preferably by one substituent R⁵. In all theses cases R⁵ is preferably selected from the group consisting of OH, oxo, C₁₋₆-alkyl, OC₁₋₆-alkyl, C(O)NH₂, C(O)OH, C(O)OC₁₋₆alkyl, C(O)NH-C₁₋₆alkyl, and C(O)N-(C₁₋₆ alkyl)₂; more preferably R⁵ is selected from the group consisting of OH, oxo, C₁₋₃-alkyl, OC₁₋₃ -alkyl, C(O)NH₂, C(O)OH, C(O)OC₁₋₃alkyl, C(O)NH-C₁₋₃alkyl, and C(O)N-(C₁₋₃alkyl)₂; and most preferably R⁵ is selected from the group consisting of OH, oxo, methyl, Omethyl, C(O)NH₂, C(O)OH, C(O)Omethyl, C(O)NH-methyl, and C(O)N-(methyl)₂.

Moreover, the present invention relates to the use of these compounds as a medicament, preferably a dermatologic agent, and as a cosmetic. The compounds are effective in the treatment and/or prevention of inflammation, irritation, itching, pruritus, pain, oedema, and/or pro-allergic or allergic conditions, particularly of the skin and mucosa of a mammal. Further conditions include hair growth (e.g. forms of alopecia, effluvium) and sebaceous gland disorders (e.g. acne, seborrhea), benign and malignant skin tumors, hyperproliferative skin diseases (e.g. psoriasis), excessive hair growth (e.g. hirsutism), different forms of dermatitis, dry skin conditions, and systemic sclerosis (scleroderma).

### Detailed Description of the Invention

In accordance with the present invention the applicant has developed the new *E*,*E*-diene compounds as defined in formula (1) and has surprisingly found out that these compounds can effectively be used as a medicament, preferably as a dermatologic agent, and as a cosmetic due to their lipophilic nature. The conditions and diseases to be treated are particularly inflammation, itching, pruritus, irritation, pain, oedema, and/or pro-allergic or allergic conditions. The compounds turned out to be effective not only in the treatment but also in the prevention of the above mentioned conditions. The conditions also comprise inflammatory reactions and irritation of skin and mucosa of a mammal, preferably of a human, e.g. caused by environmental stress and influences, such as UV irradiation, toxic substances, and allergens in general. Thus, the compounds have shown to effectively reduce several kinds of skin and mucosa discomforts as mentioned above. In particular, they have shown to provide for analgesic and anti-pruritic properties.

Without intending to be bound by any theory the applicant believes that the reason for the activity of the *E*,*E*-diene compounds of the present invention is based on their function as modulators of the endocannabinoid system. In this respect it is known that the endocannabinoids *N*-arachidonoylethanolamide (Anandamide, AEA) and 2-arachidonoylglycerol (2-AG) bind to G-protein-coupled CB₁ and CB₂ cannabinoid receptors. The CB₁ receptor is primarily (but not exclusively) expressed in neurons, the CB₂ receptor is mainly present in immunocytes, such as monocytes (splenocytes), macrophages, B cells, but also in astroglial cells. In the skin, CB receptors, in particular the CB₂ receptor, are expressed in keratinocytes and fibroblasts. Upon CB receptor activation, endocannabinoids are actively transported through the cell membrane and degraded. The metabolic enzymes fatty acid amide hydrolase (FAAH) and monoacyl glycerol lipase (MAGL) are responsible for the hydrolysis of AEA and 2-AG, respectively, thus leading to inactivation of these signaling molecules. While activation of the CB₁ receptor has been shown to mediate distinct neurophysiological effects, modulation of the CB₂ receptor by both agonists and inverse agonists is known to interfere with different inflammatory processes. CB₂ receptor activation can lead to anti-inflammatory effects *in vivo* and CB₂ receptor modulation has been implicated in the pathophysiology of a number of diseases, including chronic pain, inflammation of the gastrointestinal tract, osteoporosis, and liver diseases. Plants employ structurally similar lipid signaling molecules as animals to process cellular information, including endocannabinoid-like fatty acid derivatives. Since plants do not generally synthesize arachidonic acid, different *N*-acylethanolamines like e.g. *N*-palmitoylethanolamide are produced in plants and these lipids have also been shown to act on proteins directly or indirectly associated with the endocannabinoid system (ECS) in animals and humans, such as FAAH, the transient receptor potential vanilloid receptor (TRPV1) or the newly postulated cannabinoid receptor GPR55. Thus, in accordance with the present invention it has been found that the *E*,*E*-diene compounds of formula (1) show a significant functional interference with the ECS which is assumed to be a reason for their pharmacological activity. In particular, the claimed *E,E*-diene compounds inhibit FAAH or AEA re-uptake. In addition, some compounds of the invention inhibit FAAH in addition to AEA re-uptake. In other words, these compounds inhibit AEA reuptake or FAAH activity or both without effecting CB₁ and CB₂ receptors.

If not described in a different way herein, the employed terms for defining/describing in formula (1) the respective groups R (i.e. R¹ to R⁵) including their substituents, if any, in accordance with the present invention have the meaning as described below:
Alkyl is a straight chain or branched alkyl having 1, 2, 3, 4, 5 or 6 carbon atoms, such as methyl, ethyl, n-propyl, isopropyl, n-butyl, isobutyl, tert-butyl, n-pentyl, isopentyl, neopentyl, or hexyl.

Alkenyl is a straight chain or branched alkyl having 2, 3, 4, 5 or 6 carbon atoms and one to three double bonds, preferably one or two double bonds, most preferably one double bond. Preferred examples of a C₂₋₆alkenyl group are ethenyl, prop-1-enyl, pro-2-enyl, isoprop-1-enyl, n-but-1-enyl, n-but-2-enyl, n-but-3-enyl, isobut-1-enyl, isobut-2-enyl, n-pent-1-enyl, n-pent-2-enyl, n-pent-3-enyl, n-pent-4-enyl, n-pent-1,3-enyl, isopent-1-enyl, isopent-2-enyl, neopent-1-enyl, n-hex-1-enyl, n-hex-2-enyl, n-hex-3-enyl, n-hex-4-enyl, n-hex-5-enyl, n-hex-1,3-enyl, n-hex-2,4-enyl, n-hex-3,5-enyl, and n-hex-1,3,5-enyl. More preferred examples of a C₂₋₆alkenyl group are ethenyl and prop-1-enyl.

Alkinyl is a straight chain or branched alkyl having 2, 3, 4, 5 or 6 carbon atoms and one to three triple bonds, preferably one or two triple bonds, most preferably one triple bond. Preferred examples of a C₂₋₆alkinyl group are ethinyl, prop-1-inyl, pro-2-inyl, n-but-1-inyl, n-but-2-inyl, n-but-3-inyl, n-pent-1-inyl, n-pent-2-inyl, n-pent-3-inyl, n-pent-4-inyl, n-pent-1,3-inyl, isopent-1-inyl, neopent-1-inyl, n-hex-1-inyl, n-hex-2-inyl, n-hex-3-inyl, n-hex-4-inyl, n-hex-5-inyl, n-hex-1,3-inyl, n-hex-2,4-inyl, n-hex-3,5-inyl, and n-hex-1,3,5-inyl. More preferred examples of a C₂₋₆alkinyl group are ethinyl and prop-1-inyl.

Cycloalkyl is an alkyl ring having 3, 4, 5, 6 or 7 carbon atoms at the most, such as cyclopropyl, cyclobutyl, cyclopentyl, cyclohexyl or cycloheptyl, more preferably 3, 4, 5 or 6 carbon atoms.

Rings are saturated or mono- to polyunsaturated carbon ring systems which may include one or more heteroatoms, such as O, N, S, and/or P. Preferred are 5- or 6-membered rings. Rings particularly include aryl and heteroaryl rings, which may be fused aromatic rings.

Aryl is an aromatic moiety having 6 to 20 carbon atoms, preferably 6 to 10 carbon atoms, and includes fused aromatic rings. Most preferred is phenyl.

Heteroaryl is an aromatic moiety having 1, 2, 3, 4, 5, 6, 7, 8 or 9 carbon atoms and at least one heteroatom selected from O, N and/or S. Preferably, heteroaryl comprises at most 10 ring atoms (including carbon and heteroatoms) and is preferably selected from thienyl, pyrrolyl, imidazolyl, pyrazolyl, pyridyl, pyrazinyl, pyrimidinyl, pyridazinyl, thiazolyl, oxazolyl, isothiazolyl, isoxazolyl, furanyl, oxadiazolyl, thiadiazolyl, tetrazolyl and indazolyl, more preferably from thienyl, furanyl, imidazolyl, pyridyl, thiazolyl, oxazolyl, oxadiazolyl, thiadiazolyl, tetrazolyl and pyrimidinyl.

Heterocyclyl is a saturated or unsaturated ring containing at least one heteroatom selected from O, N and/or S and 1, 2, 3, 4, 5, 6 or 7 carbon atoms. Preferably, heterocyclyl comprises at most 11 ring atoms (including carbon and heteroatoms) and more preferably is a 4 to 8-membered ring and even more preferably is selected from tetrahydrofuranyl, azetidinyl, pyrrolidinyl, piperidinyl, pyranyl, morpholinyl, thiazolinyl, dioxanyl, dioxolanyl, and thiomorpholinyl, more preferably from piperidinyl, thiazolinyl, dioxanyl, dioxolanyl, and pyrrolidinyl.

Halogen is a halogen atom selected from F, Cl, Br and I, preferably from F, Cl and Br.

The compounds of the present invention contain a *E*, *E*-diene basic structure including an ether functionality and are represented by the following formula (1).

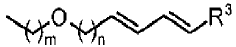

In formula (1) the residue R³ is selected from the group consisting of the following four members: -CH₂N(R⁴)C(O)R¹, -CH²N(R⁴)C(O)NR¹R², -CH₂N(R⁴)C(O)OR¹ and -C(O)NR¹R². Preferably R³ is -CH₂N(R⁴)C(O)NR¹R² or -CH₂N(R⁴)C(O)OR¹. Most preferably in formula (1) R³ is -CH₂N(R⁴)C(O)OR¹.

In all these definitions the residue R⁴ is either hydrogen or C₁₋₆-alkyl, such as C₁₋₃-alkyl (namely methyl, ethyl, n- or iso-propyl). Preferably R⁴ is either hydrogen or methyl.

Independently of the definition of R³ as mentioned above, n is defined as 1, 2, 3, 4, or 5; m is defined as 0, 1, 2, 3, or 4; and m + n is defined as 1, 2, 3, 4, 5, 6 or 7. Preferably n is defined as 1, 2, or 3, more preferably as 1 or 2, and most preferably as 2; m is defined as 2, 3, or 4, more preferably as 3 or 4, and most preferably as 3; and m + n is defined as 3, 4, 5, 6 or 7, more preferably as 4, 5, or 6, and most preferably as 5.

In all definitions of R³ according to the invention the residues R¹ and R² are independently selected from hydrogen, C₁₋₆ alkyl, C₃₋₇ cycloalkyl, C₆₋₁₀ aryl, 5- to 10-membered heteroaryl, (C₆₋₁₀ aryl)-C₁₋₆ alkyl, (5- to 10-membered heteroaryl)-C₁₋₆ alkyl, (C₆₋₁₀ aryl fused with a 4 to 8-membered saturated or unsaturated heterocyclyl containing 1 to 3 heteroatoms independently selected from N, O, S), (C₆₋₁₀ aryl fused with a 4 to 8-membered saturated or unsaturated heterocyclyl containing 1 to 3 heteroatoms independently selected from N, O, S)-C₁₋₆alkyl, C₁₋₆alkyl-C(O)OC₁₋₆alkyl, C₁₋₆alkyl-C(O)NH(C₁₋₆alkyl), C₁₋₆alkyl-C(O)N(C₁₋₆ alkyl)₂, C₁₋₆alkyl-CN.

More preferably in the compounds of formula (1), that contain -NR¹R² moieties in the definition of R³, one of R¹ and R² is hydrogen and the other is selected from (C₆₋₁₀ aryl)-C₁₋₆ alkyl, (5- to 10-membered heteroaryl)-C₁₋₆ alkyl, (C₆₋₁₀ aryl fused with a 4 to 8-membered saturated or unsaturated heterocyclyl containing 1 to 3 heteroatoms independently selected from N, O, S), and (C₆₋₁₀ aryl fused with a 4 to 8-membered saturated or unsaturated heterocyclyl containing 1 to 3 heteroatoms independently selected from N, O, S)-C₁₋₆alkyl.

Further in the compounds of formula (1), that contain -C(O)R¹ or -C(O)OR¹ moieties in the definition of R³, R¹ is preferably selected from C₆₋₁₀ aryl, 5- to 10-membered heteroaryl, (C₆₋₁₀ aryl)-C₁₋₆ alkyl, (5- to 10-membered heteroaryl)-C₁₋₆ alkyl, (C₆₋₁₀ aryl fused with a 4 to 8-membered saturated or unsaturated heterocyclyl containing 1 to 3 heteroatoms independently selected from N, O, S), (C₆₋₁₀ aryl fused with a 4 to 8-membered saturated or unsaturated heterocyclyl containing 1 to 3 heteroatoms independently selected from N, O, S)-C₁₋₆alkyl, C₁₋₆alkyl-C(O)OC₁₋₆alkyl, C₁₋₆alkyl-C(O)NH(C₁₋₆alkyl), C₁₋₆alkyl-C(O)N(C₁₋₆alkyl)₂, and C₁₋₆ alkyl-CN. More preferably R¹ is selected from C₆₋₁₀ aryl, (C₆₋₁₀ aryl)-C₁₋₆ alkyl, C₁₋₆alkyl-CN, C₁₋₆alkyl-C(O)NH(C₁₋₆alkyl), C₁₋₆alkyl-C(O)N(C₁₋₆alkyl)₂, and (5- to 10-membered heteroaryl)-C₁₋₆ alkyl.

In formula (1) R¹, R², and R⁴, independently of each other, may be substituted by one, two or three substituents R⁵, preferably by one or two substituents R⁵, more preferably by one substituent R⁵. However, R⁴ most preferably is not substituted at all.

In accordance with the present invention the substituent R⁵ is selected from the group consisting of halogen; CN; OH; oxo; C₁₋₆-alkyl, optionally substituted with one or more substituents selected from halogen, OH, OC₁₋₆alkyl, OC₃₋₇cycloalkyl; OC₁₋₆-alkyl, optionally substituted with one or more substituents selected from halogen, OH, OC₁₋₆alkyl, OC₃₋₇ cycloalkyl; C₆₋₁₀-aryl; 5- to 10-membered heteroaryl; 5- to 11-membered heterocyclyl, optionally substituted with one or more substituents selected from halogen, OH, oxo, OC₁₋₆ alkyl, OC₃₋₇cycloalkyl; C(O)NH₂; C(O)OH; C(O)OC₁₋₆alkyl, optionally substituted with one or more substituents selected from halogen, OH, OC₁₋₆alkyl, OC₃₋₇cycloalkyl; C(O)OC₃₋₇ cycloalkyl, optionally substituted with one or more substituents selected from halogen, OH, OC₁₋₆alkyl, OC₃₋₇cycloalkyl; C(O)NH-C₁₋₆alkyl, optionally substituted with one or more substituents selected from halogen, OH, OC₁₋₆alkyl, OC₃₋₇cycloalkyl; C(O)NH-C₃₋₇ cycloalkyl, optionally substituted with one or more substituents selected from halogen, OH, OC₁₋₆alkyl, OC₃₋₇cycloalkyl; C(O)N-(C₁₋₆alkyl)₂, optionally substituted with one or more substituents selected from halogen, OH, OC₁₋₆alkyl, OC₃₋₇cycloalkyl; C(O)N-(C₁₋₆alkyl)(C₃₋₇ cycloalkyl), optionally substituted with one or more substituents selected from halogen, OH, OC₁₋₆alkyl, OC₃₋₇cycloalkyl; SO₂-C₁₋₆alkyl, optionally substituted with one or more substituents selected from halogen, OH, OC₁₋₆alkyl, OC₃₋₇cycloalkyl; NH₂; NH-C₁₋₆alkyl, optionally substituted with one or more substituents selected from halogen, OH, OC₁₋₆alkyl, OC₃₋₇cycloalkyl; N-(C₁₋₆alkyl)₂, optionally substituted with one or more substituents selected from halogen, OH, OC₁₋₆alkyl, OC₃₋₇cycloalkyl; NH-SO₂-CH₃; NH-C(O)OC₁₋₆alkyl, optionally substituted with one or more substituents selected from halogen, OH, OC₁₋₆alkyl, OC₃₋₇cycloalkyl; NH-C(O)OC₃₋₇cycloalkyl, optionally substituted with one or more substituents selected from halogen, OH, OC₁₋₆alkyl, OC₃₋₇cycloalkyl; N(C₁₋₆alkyl)-C(O)OC₁₋₆ alkyl, optionally substituted with one or more substituents selected from halogen, OH, OC₁₋₆ alkyl, OC₃₋₇cycloalkyl; N(C₁₋₆alkyl)-C(O)OC₃₋₇cycloalkyl, optionally substituted with one or more substituents selected from halogen, OH, OC₁₋₆alkyl, OC₃₋₇cycloalkyl; NH-C(O)C₁₋₆ alkyl, optionally substituted with one or more substituents selected from halogen, OH, OC₁₋₆ alkyl, OC₃₋₇cycloalkyl; NH-C(O)C₃₋₇cycloalkyl, optionally substituted with one or more substituents selected from halogen, OH, OC₁₋₆alkyl, OC₃₋₇cycloalkyl; N(C₁₋₆alkyl)-C(O)C₁₋₆ alkyl, optionally substituted with one or more substituents selected from halogen, OH, OC₁₋₆ alkyl, OC₃₋₇cycloalkyl; and N(C₁₋₆alkyl)-C(O)C₃₋₇cycloalkyl, optionally substituted with one or more substituents selected from halogen, OH, OC₁₋₆alkyl, OC₃₋₇cycloalkyl.

In the above mentioned definitions of formula (1) R¹ and R², independently of each other, preferably are substituted by one or two substituents R⁵, more preferably by one substituent R⁵. In all theses cases R⁵ is preferably selected from the group consisting of OH, oxo, C₁₋₆-alkyl, OC₁₋₆-alkyl, C(O)NH₂, C(O)OH, C(O)OC₁₋₆alkyl, C(O)NH-C₁₋₆alkyl, and C(O)N-(C₁₋₆ alkyl)₂; more preferably R⁵ is selected from the group consisting of OH, oxo, C₁₋₃-alkyl, OC₁₋₃ -alkyl, C(O)NH₂, C(O)OH, C(O)OC₁₋₃alkyl, C(O)NH-C₁₋₃alkyl, and C(O)N-(C₁₋₃alkyl)₂; and most preferably R⁵ is selected from the group consisting of OH, oxo, methyl, Omethyl, C(O)NH₂, C(O)OH, C(O)Omethyl, C(O)NH-methyl, and C(O)N-(methyl)₂.

All possible combinations of the definitions of R¹ to R⁵ and n and m in formula (1) included in the lists above should be understood as being directly and unambiguously disclosed according to the present invention.

In general, substituents of the carbon chains and rings in accordance with the invention are halogens, preferably F, Cl, Br and I, C₁₋₁₀ alkyl, C₁₋₁₀alkoxy, C₆₋₂₀aryl, hydroxyl, -SH, -SO₃H, amine groups, -COOH, COOR', wherein R' is a C₁₋₁₀alkyl or an alkali metal, CONHR", and CON(R")₂, wherein R" is a C₁₋₁₀alkyl. In general, particularly preferred alkyl and alkoxy groups used as substituents in accordance with the present invention as described herein are C₁₋₆alkyl and C₁₋₆alkoxy groups, more preferably methyl, ethyl, n-propyl, i-propyl, n-butyl, i-butyl, n-pentyl, iso-pentyl, methoxy, ethoxy, n-propoxy, i-propoxy, n-butoxy, i-butoxy, n-pentoxy, iso-pentoxy. Similarly, particularly preferred aryl groups are C₆₋₁₀aryl groups, most preferably phenyl. Suitable substituents are also selected from alkenyl, alkinyl, cycloalkyl, heteroaryl, heterocyclyl as defined herein.

In particularly preferred embodiments of the present invention the compounds are according to the following formulae (2) to (7):

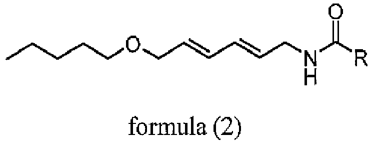

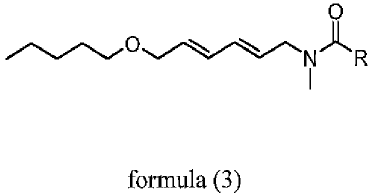

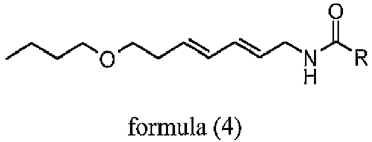

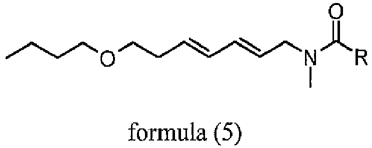

In formulae (2) to (5) R is R¹ or NR¹R² or OR¹ as defined herein.

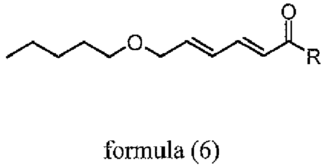

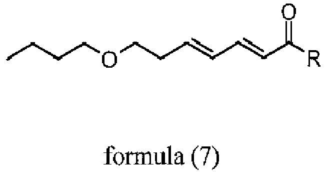

In formulae (6) and (7) R is NR¹R² as defined herein.

According to the present invention, in compounds of formulae (1) to (7), R may also be selected from the group consisting of the following radicals (which may be substituted by substituent groups as defined herein):

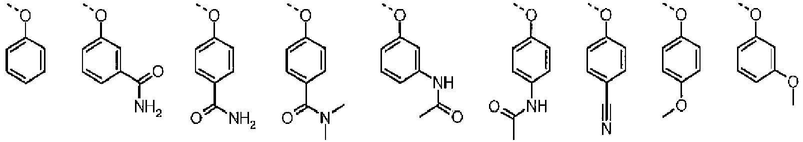

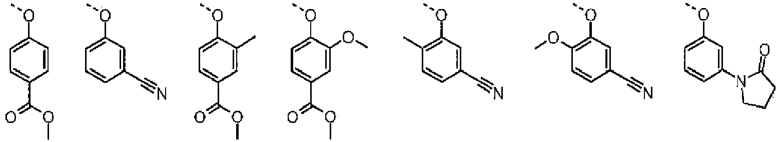

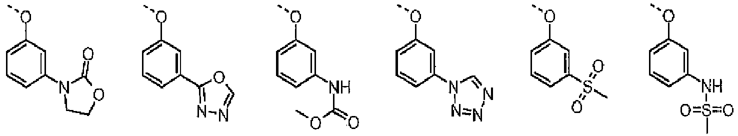

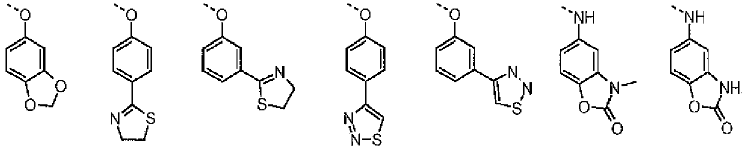

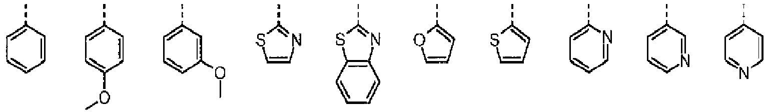

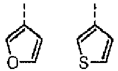

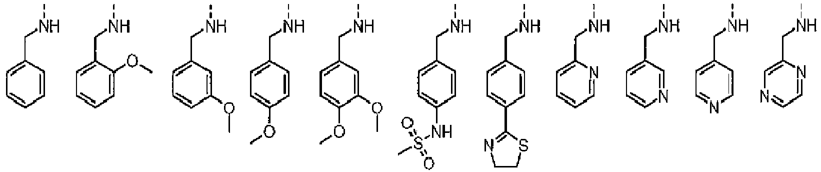

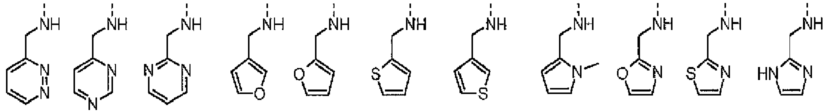

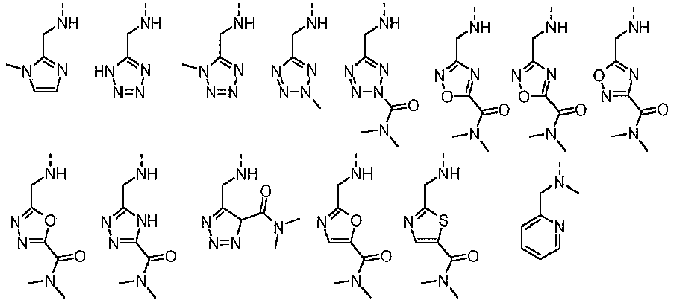

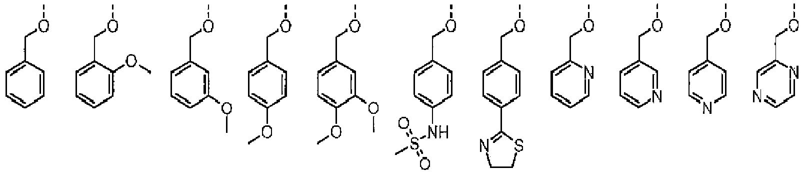

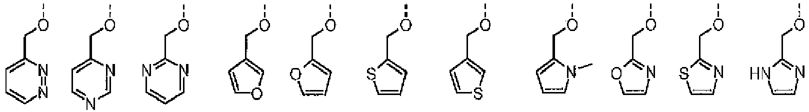

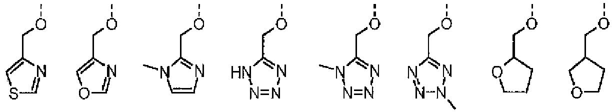

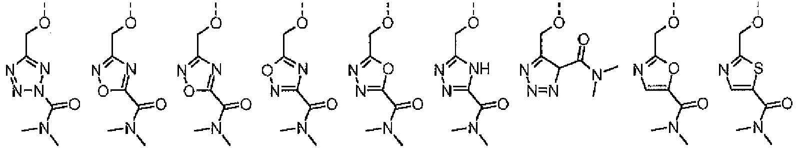

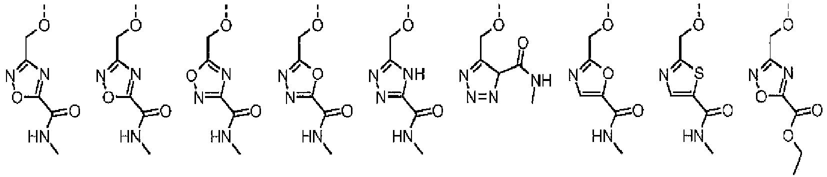

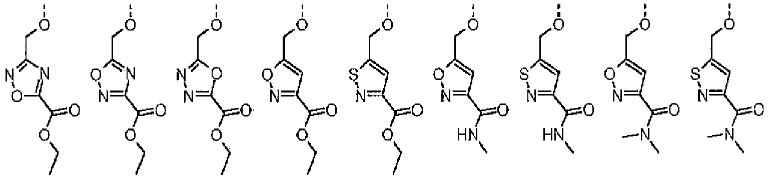

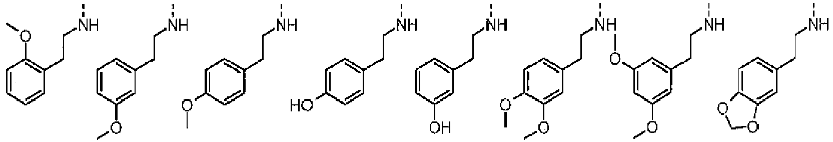

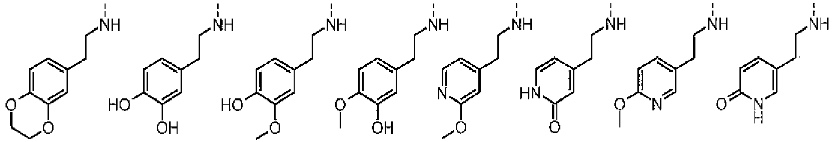

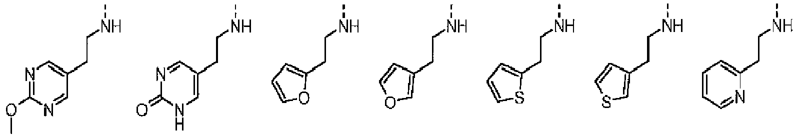

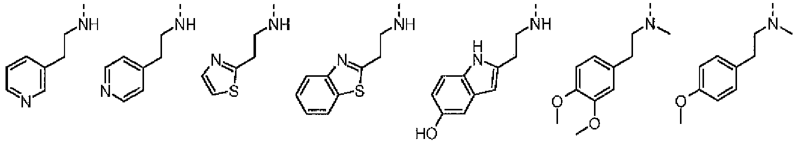

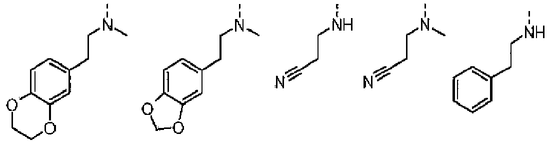

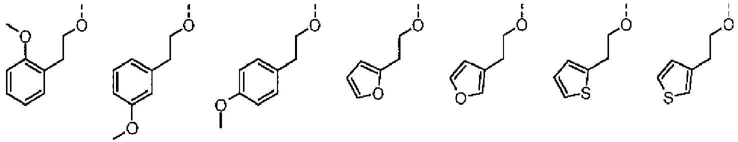

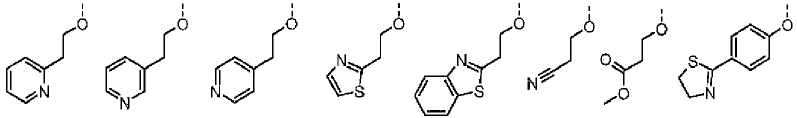

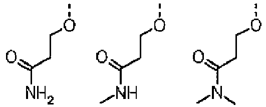

The compounds of structural formula (1) are effective as modulators of the endocannabinoid system and are particularly effective as inhibitors of AEAT (AEA transport) or FAAH. They are useful for the treatment and/or prevention of disorders responsive to inhibition of AEAT or FAAH, such as inflammation, irritation, itching, pruritus, pain, oedema and/or pro-allergic, allergic conditions and other diseases in particular with AEAT or FAAH involvement.

### Optical Isomers - Diastereomers - Geometric Isomers - Tautomers

Compounds of structural formulae (1) to (7) contain one or more asymmetric centers and can occur as racemates and racemic mixtures, single enantiomers, diastereomeric mixtures and individual diastereomers. The present invention is meant to comprehend all such isomeric forms of the compounds of structural formulae (1) to (7).

Compounds of structural formulae (1) to (7) may be separated into their individual diastereomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration. Alternatively, any stereoisomer of a compound of the general formulae (1) to (7) may be obtained by stereospecific synthesis using optically pure starting materials or reagents of known absolute configuration.

### Salts

The compounds according to the invention can be present in the form of pharmaceutical acceptable salts. The term "pharmaceutical acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N´-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, p-toluenesulfonic, trifluoroacetic acid and the like. Particularly preferred are citric, fumaric, hydrobromic, hydrochloric, maleic, phosphoric, sulfuric and tartaric acids.

### Administration, Dose Ranges and Formulation

The compounds of the invention are incorporated into pharmaceutical or cosmetic preparations by admixing them with a pharmaceutically or cosmetically acceptable carrier or excipient. Suitable carriers are known to the skilled person. Preferably the composition is a dermatological composition suitable to be applied topically on the skin or mucosa of a mammal. The form of the composition is not particularly limited, however, preferred forms are emulsions, suspensions and solutions. In certain embodiments, the compositions are in the form of lotions, creams, gels, solutions, sprays, cleansers, powders, ointments, waxes, lipsticks, soaps, shampoos, hydroalcoholic solutions, suspensions, foams, scrubs, saturated pads, skin or hair conditioning agents.

The compositions according to the invention, however, can be applied in any other way known to the skilled person such as oral or parenteral. Non-limiting examples are sublingual, vaginal, rectal, intravenous, nasal, intramuscular, inhalative, ocular and percutaneous. Suitable carriers and excipients are commonly known to the skilled person.

Compositions in accordance with the present invention may contain the diene compounds in amounts of 0.001 to 40% by weight of the composition, preferably 0.01 to 5 % by weight, more preferably 0.1 to 2 % by weight and most preferably 0.5 to 1.5 % by weight.

Compositions in accordance with the present invention may contain cosmetically and pharmaceutically/dermatologically acceptable auxiliaries usually employed in cosmetic and pharmaceutical preparations and known to the skilled person. These include for example preservatives, bactericides, perfumes, thickeners, emulsifiers, surfactants, softening agents, moisturizing agents, oils, fats, waxes, organic solvents, water, alcohols, polyols, polymers, foam stabilizers, anti-foaming agents, penetration enhancers or other conventional components of a pharmaceutical or cosmetic preparation.

### Preparation of Compounds of the Invention

The compounds of formulae (1) to (7), when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formulae (1) to (7) may be obtained by stereospecific syntheses using optically pure starting materials or reagents of known configuration.

The compounds of formulae (1) to (7) of the present invention can be prepared according to the procedures of the following schemes and examples, using appropriate materials and are further exemplified by the following specific examples. Moreover, by utilizing the procedures described herein, in conjunction with ordinary skill in the art, additional compounds of the present invention claimed herein can be readily prepared. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds. Compounds containing a basic or acidic group can be further converted into the form of their pharmaceutical acceptable salts, such as described previously. All temperatures are degrees Celsius.

In the schemes, preparations and examples below, various reagent symbols and abbreviations have the following meanings:
- AcOH: acetic acid
- Boc: tert-butoxycarbonyl
- CDI: 1,1'-carbonyldiimidazole
- DBU: 1,8-diazabicyclo[5.4.0]undec-7-ene
- DCM: dichloromethane
- DiBALH: diisobutylaluminum hydride
- DIEA: ethyl-diisopropylamine
- DMAP: 4-dimethylaminopyridine
- DMF: N,N-dimethylformamide
- DPPA: diphenyl phosphoryl azide
- EDCI: N-(3-dimethylaminopropyl)-N'-ethylcarbodiimide
- Et₂O: diethyl ether
- EtOH: ethanol
- h: hour(s)
- HATU: O-(7-azabenzotriazol-1-yl)-N,N,N',N'-tetramethyluronium hexafluorophosphate
- HOAt: 1-hydroxy-7-azabenzotriazole
- m/z: mass-to-charge ratio
- min: minute(s)
- MeOH: methanol
- mp: melting point
- MW: molecular weight
- Ph: phenyl
- RT: room temperature
- TEA: triethylamine
- THF: tetrahydrofuran
- TLC: thin layer chromatography
- t_{R} (min): HPLC retention time

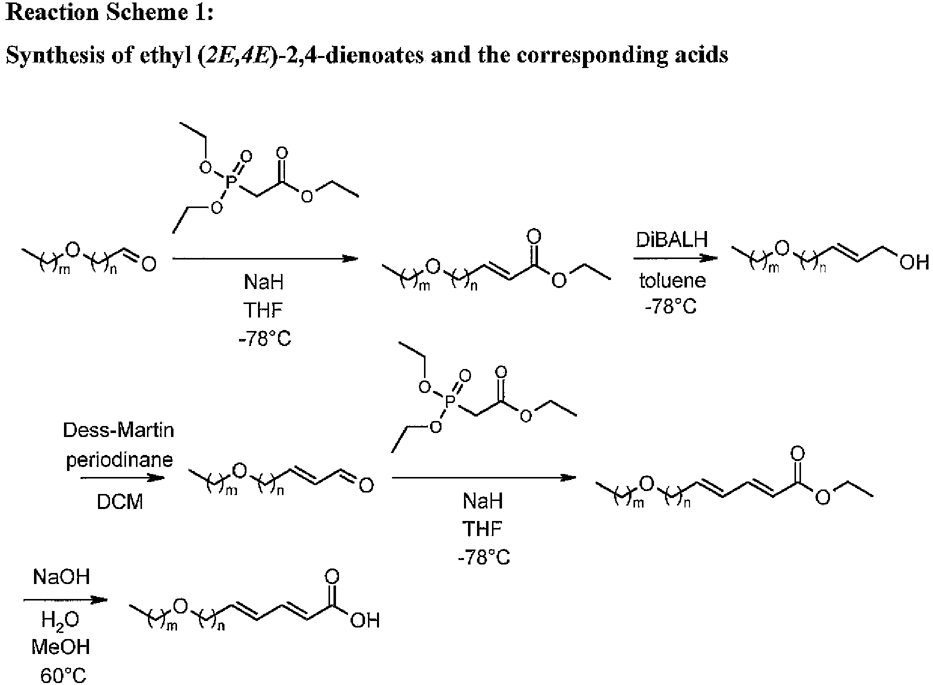

As shown in Reaction Scheme 1, the starting material for the synthesis of compounds of the present invention, ethyl (*2E*,*4E*)-2,4-dienoates and (*2E*,*4E*)-2,4-dienoic acids can be prepared starting from saturated aldehydes. The two double bonds with *trans* stereochemistry are introduced applying two Wittig-Horner reactions. α,β-Unsaturated ester is obtained by reaction of an aldehyde with trialkylphosphonoacetate in an inert solvent like tetrahydrofuran in the presence of a base such as sodium hydride or butyl lithium at low temperature. Reduction with DiBALH in a suitable solvent like toluene at -78°C provides the corresponding allylic alcohol which is subsequently oxidized with Dess-Martin periodinane in an appropriate solvent such as dichloromethane to the α,β-unsaturated aldehyde. Alternatively, the alcohol can be oxidized applying a Swern oxidation using oxalyl chloride and DMSO in an appropriate solvent like DCM in the presence of a base such as triethylamine at -78°C. The aldehyde is subjected to a second Wittig-Horner reaction applying the conditions described above to yield the ethyl (*2E*,*4E*)-2,4-dienoate. Finally, (2E,4E)-2,4-dienoic acid is obtained by hydrolyzing the ester in the presence of a base such as aqueous sodium hydroxide in methanol at elevated temperature.

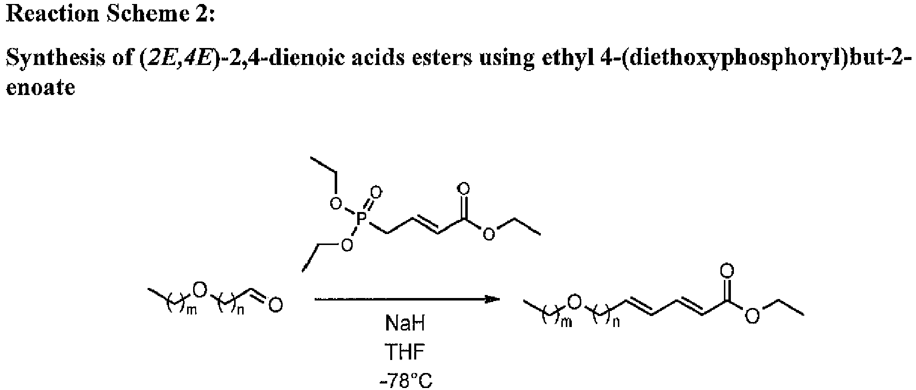

Another possibility for the synthesis of ethyl (*2E*,*4E*)-2,4-dienoates is depicted in Reaction Scheme 2. Saturated aldehydes can be reacted with ethyl 4-(diethoxyphosphoryl)but-2-enoate in the presence of a base such as sodium hydride in a solvent like tetrahydrofuran at low temperature to yield the target compound.

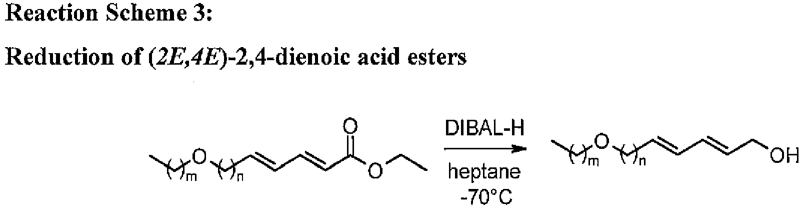

(2E, *4E*)-2,4-Dienoic acid ester can be reduced to the corresponding alcohol by reaction with a reducing reagent such as DiBALH in an inert solvent like heptane at a suitable temperature, as depicted in Reaction Scheme 3.

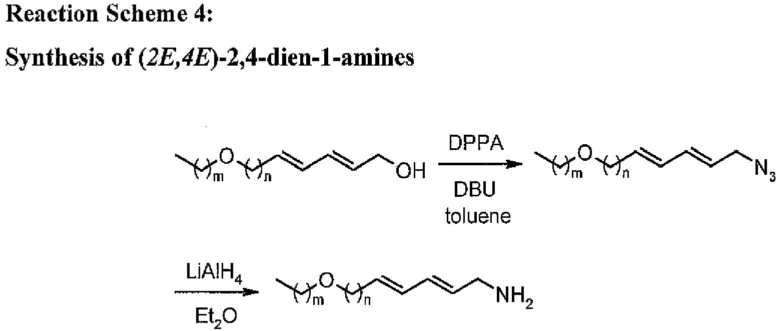

Reaction Scheme 4 shows the preparation of (*2E*,*4E*)-2,4-dien-1-amines. (*2E*,*4E*)-2,4-Dien-1-ylalcohol can be reacted with a reagent such as diphenyl phosphoryl azide in the presence of an appropriate base like 1,8-diazabicyclo[5.4.0]undec-7-ene in a solvent such as toluene to yield the corresponding azide. Subsequent reduction with a reducing agent like lithium aluminum hydride in an inert solvent such as diethyl ether at an appropriate temperature provides the target compound.

Alternatively, the azide can be reduced to the corresponding primary amine in a Staudinger reaction by reaction with triphenylphosphine in a suitable solvent such as a mixture of water and THF.

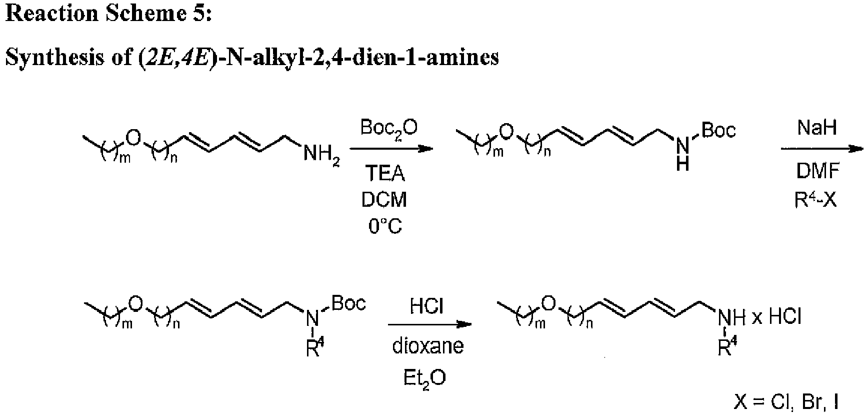

(*2E*,*4E*)-2,4-Dien-1-ylamine can be converted to the corresponding N-alkylated amine as depicted in Reaction Scheme 5. Reaction with di-tert-butyl dicarbonate in the presence of a base like triethylamine in a suitable solvent like DCM is followed by an alkylation step with an alkyl halogenide like methyl iodide in a solvent like DMF in the presence of a base such as sodium hydride. The alkylated amine can be deprotected using a reagent like HCl in dioxane in an appropriate solvent like diethyl ether. The product can be isolated as hydrochloride salt or the free base can be liberated.

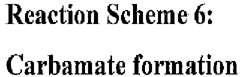

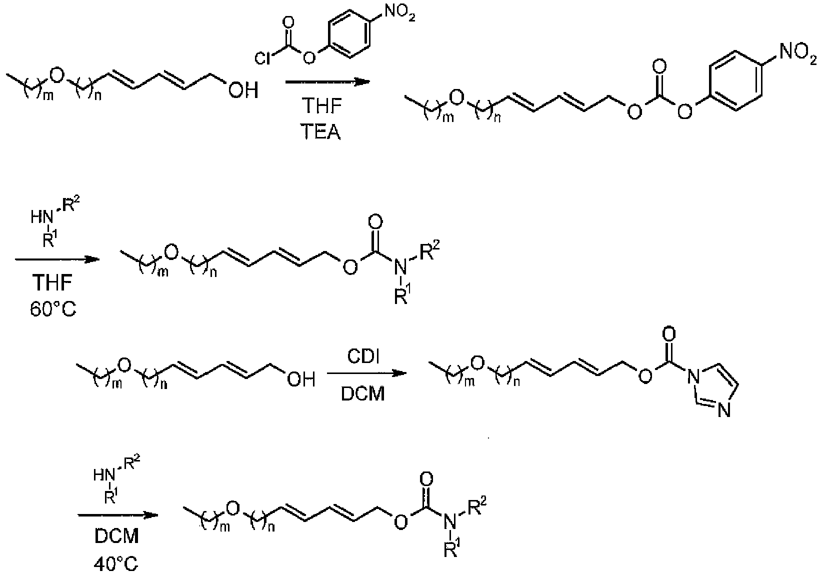

Reaction Scheme 6 shows the synthesis of carbamates. (*2E*,*4E*)-2,4-Dien-1-ylalcohol can be activated with a reagent such as p-nitrophenylchloroformate in the presence of a base like triethylamine in a solvent such as tetrahydrofuran. Alternatively, activation can be accomplished by reaction with 1,1'-carbonyldiimidazole in a solvent like dichloromethane. Another option for activating (*2E*,*4E*)-2,4-dien-1-ylalcohol is the reaction with triphosgene in an inert solvent in the presence of a base such as triethylamine. Subsequent reaction with optionally substituted amines in solvents like dichloromethane or tetrahydrofuran at appropriate temperatures provides the target compounds.

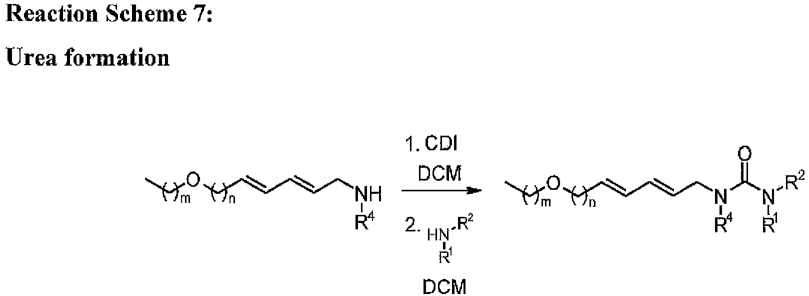

Reaction Scheme 7 shows the formation of optionally substituted (*2E*,*4E*)-2,4-dien-1-amine-derived urea by activation with 1,1'-carbonyldiimidazole in an inert solvent like dichloromethane and subsequent reaction with optionally substituted amine HNR¹R².

Alternatively, the reaction can also be performed using triphosgene as reagent in the presence of a base such as triethylamine.

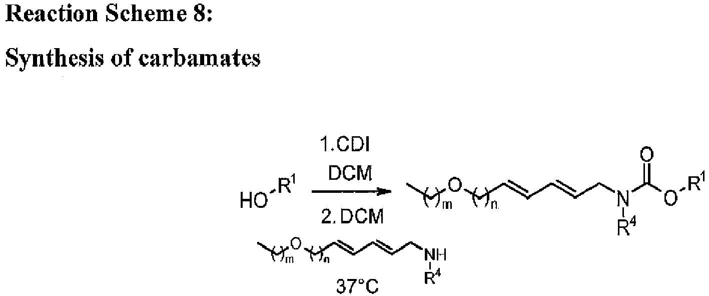

Reaction Scheme 8 shows the synthesis of carbamates. Optionally substituted alcohol HO-R¹ is activated with a reagent such as 1,1'-carbonyldiimidazole in an inert solvent like DCM. Subsequent reaction with optionally substituted (*2E*,*4E*)-2,4-dien-1-amine at a suitable temperature yields the target compound.

Alternatively, alcohol HO-R¹ can also be activated using p-nitrophenylchloroformate in an inert solvent like THF in the presence of a suitable base like triethylamine at an appropriate temperature.

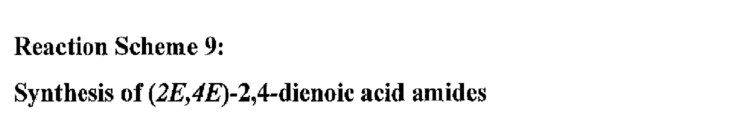

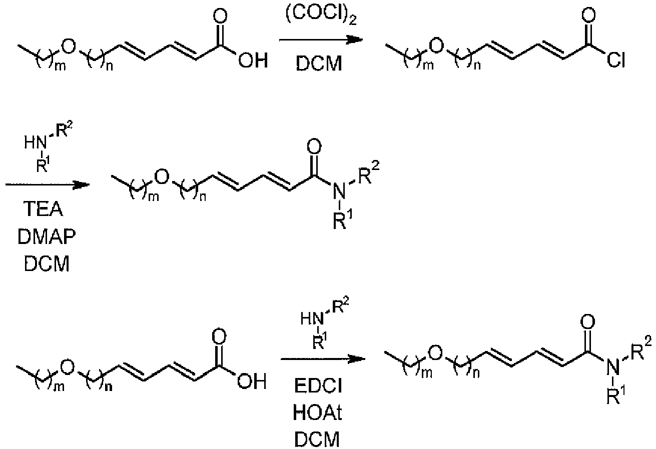

(*2E*,*4E*)-2,4-Dienoic acid can be converted to the corresponding acid chloride by reaction with a reagent such as oxalyl chloride or thionyl chloride in a suitable solvent like DCM. Reaction with an amine HNR¹R² in an inert solvent like DCM yields the desired product. The reaction can be carried out in the presence or without a base such as DMAP.

Alternatively, synthesis of optionally substituted (*2E*,*4E*)-2,4-dienoic acid amides can also be accomplished by reacting the corresponding acid with an amine HNR¹R² in a solvent like DCM or DMF in the presence of a reagent such as HOAt and EDCI.

### Analytical LC-MS

Analytical conditions summary:
Agilent 1100 with ELSD (PL-ELS 2100) and diode array detector with UV-detection between 220 and 320 nm and Mass Selective Detector (MSD) in ESI+ and ESI- modus (mass range: m/z= 100-800),
Column: Waters Xbridge C18, 3.5 µm, 2.1 mm x 50 mm;
Flow rate of 0.8 ml/min; column temperature: 30°C;

| | |
|---|---|
| Mobile Phase A: | acetonitrile (0.1 % HCOOH) |
| Mobile Phase B: | water (0.1% HCOOH) |

or
Agilent 1200 with diode array detector with UV-detection between 220 and 320 nm and Mass Selective Detector (MSD) in ESI+ and ESI- modus (mass range: m/z = 100-800),
Column: Waters Xbridge C18, 3.5 µm, 2.1 mm x 50 mm;
Flow rate of 0.8 ml/min; column temperature: 30°C;

| | |
|---|---|
| Mobile Phase A: | acetonitrile (0.1% HCOOH) |
| Mobile Phase B: | water (0.1% HCOOH) |

### Gradient A:

### linear gradient from 2% to 98% acetonitrile in water (0.1% HCOOH)

| | |
|---|---|
| 0.0 min | 2% A |
| 3.5 min | 98% A |
| 6.0 min | 98% A |

Agilent 1100 with ELSD (PL-ELS 2100) and diode array detector (Agilent G1315B) with UV-detection between 220 and 320 nm and Mass Selective Detector (MSD, Agilent LC/MSD G6130B) in ESI+ and ESI- modus (mass range: m/z = 100-800),
Column: Waters Xbridge C18, 3.5 µm, 2.1 mm x 50 mm;
Flow rate of 0.8 ml/min; column temperature: 30°C;
Mobile Phase A: 95% acetonitrile + 5% 10 mM ammonium bicarbonate in water
Mobile Phase B: 10 mM ammonium bicarbonate in water (pH = 9.5)

### Gradient B:

linear gradient from 2% to 98% of 95% acetonitrile + 5% 10 mM ammonium bicarbonate in water

| | |
|---|---|
| 0.0 min | 2% A |
| 3.5 min | 98% A |
| 6.0 min | 98% A |

Column: Waters Xselect C18, 3.5 µm, 2.1 mm x 50 mm;
Flow rate of 0.8 ml/min; column temperature: 25°C;
Mobile Phase A: 95% acetonitrile + 5% 10 mM ammonium bicarbonate in water
Mobile Phase B: 10 mM ammonium bicarbonate in water (pH =9.5)

### Gradient C:

### linear gradient from 2% to 98% of 95% acetonitrile + 5% 10 mM ammonium bicarbonate in water

| | |
|---|---|
| 0.0 min | 2% A |
| 3.5 min | 98% A |
| 8.0 min | 98% A |

The following describes the detailed examples of the invention which have been prepared via reaction schemes 1 to 9.

**Table 1:**

| | | | | | | | |
|---|---|---|---|---|---|---|---|
| 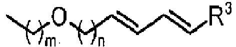 | | | | | | | |

| | | | | HPLC | | MS | |
|---|---|---|---|---|---|---|---|
| No. | m | n | R³ | t_{R} (min) | method | MW (calc.) free base | [M+H⁺] (found) |
| 1 | 4 | 1 | 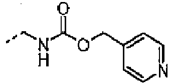 | 3.67 | B | 318.42 | 319 |
| 2 | 4 | 1 | 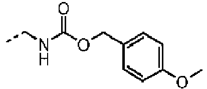 | 4.12 | B | 347.46 | 365¹⁾ |
| 3 | 4 | 1 | 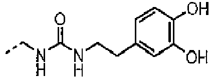 | 3.37 | A | 362.47 | 363 |
| 4 | 4 | 1 | 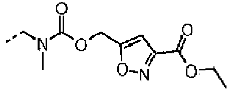 | 4.25 | B | 394.47 | 412¹⁾ |
| 5 | 4 | 1 | 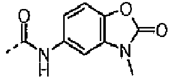 | 3.78 | B | 344.41 | 345 |
| 6 | 3 | 2 | 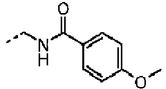 | 3.69 | A | 317.43 | 318 |
| 7 | 3 | 2 | 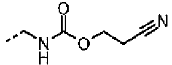 | 3.53 | A | 280.37 | 281 |
| 8 | 3 | 2 | 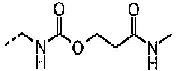 | 3.36 | B | 312.41 | 313 |
| 9 | 3 | 2 | 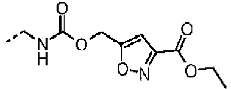 | 4.01 | B | 380.44 | 381 |
| 10 | 3 | 2 | 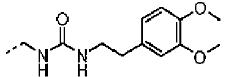 | 3.57 | A | 390.53 | 391 |
| 11 | 3 | 2 | 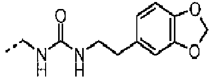 | 3.76 | A | 374.48 | 375 |
| 12 | 3 | 2 | 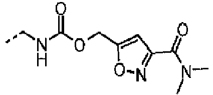 | 3.56 | A | 379.46 | 380 |
| 13 | 3 | 2 | 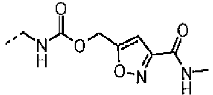 | 3.38 | B | 365.43 | 366 |
| 14 | 3 | 2 | 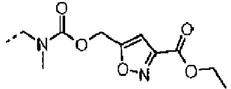 | 4.03 | A | 394.47 | 395 |
| 15 | 3 | 2 | 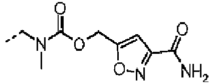 | 3.73 | B | 365.43 | 366 |
| 16 | 3 | 2 | 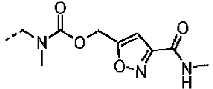 | 3.83 | B | 379.46 | 380 |
| 17 | 3 | 2 | 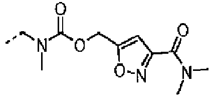 | 3.92 | B | 393.49 | 394 |
| 18 | 3 | 2 | 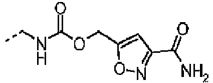 | 3.58 | C | 351.41 | 352 |

| | | | | | | | |
|---|---|---|---|---|---|---|---|
| ¹⁾[M+NH₃ + H]⁺ | | | | | | | |

The following examples are provided to illustrate the invention and are not limiting the scope of the invention in any manner.

### Common Intermediates:

### 1-(2,2-Diethoxyethoxy)pentane

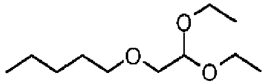

Sodium hydride (60% wt., 4.06 g) was added to a cooled solution of pentan-1-ol (11.03 ml) in dry tetrahydrofuran (100 ml). Bromoacetaldehyde diethyl acetal (17.19 ml) was added and the mixture was stirred overnight at reflux. The reaction mixture was allowed to cool to room temperature and concentrated *in vacuo.* The residue was treated with water and extracted with diethyl ether. The combined organic layer was washed with brine and dried over Na₂SO₄, concentrated *in vacuo* (300 mbar) affording the diacetal (purity 69%). The product was used as such in the next step.

### (Pentyloxy)acetaldehyde

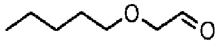

To a solution of 1-(2,2-diethoxyethoxy)pentane (22.1 g,) in 1,4-dioxane (100 ml) and water (150 ml) was added sulfuric acid (6.05 ml). The mixture was stirred at 80°C for 2 h. The reaction mixture was allowed to cool to room temperature and poured into ∼700 ml saturated NaHCO₃ (aq) (pH∼7). The product was extracted with three times with CH₂Cl₂. The combined organic layer was washed with brine, dried over Na₂SO₄ and concentrated *in vacuo* (30°C). The crude product was used as such in the next step (purity approximately 40%).

### Ethyl (2E-4-{pentyloxy)but-2-enoate

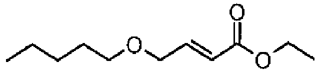

Sodium hydride (60% wt., 1.784 g) was suspended in dry tetrahydrofuran (100 ml) and cooled to 0°C. Triethyl phosphonoacetate (8.85 ml) was added dropwise. The reaction mixture was stirred at 0°C for 30 min, cooled to -78°C after which 2-(pentyloxy)acetaldehyde (12.1 g, 40% purity) in dry tetrahydrofuran (5 ml) was added dropwise. The reaction mixture was stirred at this temperature for 1 h. The reaction mixture was allowed to warm to room temperature, hydrolyzed with water/THF, poured into water and the product was extracted twice with EtOAc. The combined organic layer was washed with brine, dried over Na₂SO₄, and concentrated *in vacuo.* The crude material was purified via column chromatography (10% EtOAc in heptane) to yield the product as a colorless oil.

### (2E)-4-(Pentyloxy)but-2-en-1-ol

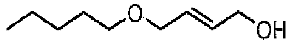

A solution of (*E*)-ethyl 4-(pentyloxy)but-2-enoate (6.28 g) in dry toluene (75 ml) was cooled to -78°C. DiBALH (1M in hexanes, 64.3 ml) was added dropwise and the mixture was stirred for 2.5 h. The mixture was allowed to warm to room temperature and hydrolyzed with 1M HCl (aq) (cooling in ice/water bath). Additional 3M HCl (aq) was added until pH=1 and the aqueous layer was extracted three times with EtOAc. The combined organic layer was washed with brine, dried over Na₂SO₄ and concentrated *in vacuo* to yield the alcohol, which was used as such in the next step.

### (2E)-4-(Pentyloxy)but-2-enal

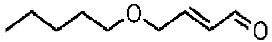

(*E*)-4-(pentyloxy)but-2-en-1-ol (5.02 g) was dissolved in dichloromethane (50 ml), Dess-Martin periodinane (13.91 g) was added portionwise and the mixture was stirred at room temperature for 1 h. The reaction mixture was hydrolyzed with saturated NaHCO₃ (aq). The aqueous layer (white suspension) was extracted twice with CH₂Cl₂. The combined organic layer was washed with brine, dried over Na₂SO₄ and concentrated *in vacuo.* The concentrate was dissolved in a minimum amount of CH₂Cl₂ and Et₂O was added (suspension). The solids were filtered off (glass filter) and the filtrate was concentrated *in vacuo* to yield the aldehyde. Due to the possible instability of the product, no purifications were performed.

### Ethyl (2E,4E)-6-(pentyloxy)hexa-2,4-dienoate

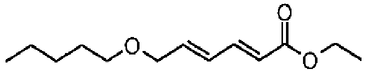

Sodium hydride (60% wt., 1.270 g) was suspended in dry tetrahydrofuran (50 ml) and cooled to 0°C. Triethyl phosphonoacetate (6.30 ml) was added dropwise. The reaction mixture was stirred at 0°C for 30 min, cooled to -78°C after which (*E*)-4-(pentyloxy)but-2-enal (4.35 g) in dry tetrahydrofuran (1 ml) was added dropwise. The reaction mixture was stirred at this temperature for 1 h. The reaction mixture was allowed to warm to room temperature, hydrolyzed with water/THF, poured into water and the product was extracted twice with EtOAc. The combined organic layer was washed with brine, dried over Na₂SO₄, and concentrated *in vacuo.* The crude material was purified via column chromatography (10% EtOAc in heptane) affording the desired product as a colorless oil.

### (2E,4E)-6-(Pentyloxy)hexa-2,4-dien-1-ol

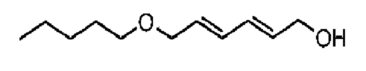

A solution of (*2E*,*4E*)-ethyl 6-(pentyloxy)hexa-2,4-dienoate (3.18 g) in dry toluene (35 ml) was cooled to -78°C. DiBALH (1M in hexanes, 28.8 ml) was added dropwise and the mixture was stirred for 2.5 h at -78°C. The mixture was allowed to warm to room temperature and hydrolyzed with 1M HCl (aq) (cooling in ice/water bath). Additional, 3M HCl (aq) was added until pH=1. The layers were separated and the aqueous layer was extracted three times with EtOAc. The combined organic layer was washed with brine, dried over Na₂SO₄ and concentrated *in vacuo.* The crude material was purified via column chromatography (10-50% EtOAc in heptane) to yield the desired alcohol as a colorless oil.

### (2E,4E)-6-Azidohexa-2,4-dien-1-yl pentyl ether

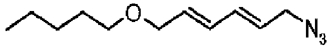

(*2E*,*4E)*-6-(pentyloxy)hexa-2,4-dien-1-ol (1.94 g) was dissolved in dry toluene (25 ml), cooled in an ice bath and diphenyl phosphorazidate (2.281 ml) was added. DBU (1.887 ml) was added dropwise. The reaction mixture was stirred at room temperature overnight. The solvent was evaporated and the residue was partitioned between diethyl ether and 1 N NaOH. The organic layer was separated and washed with 1 M HCl, water and brine, dried over Na₂SO₄ and concentrated *in vacuo.* The crude product was purified by flash column chromatography (10% EtOAc in heptane) to yield the azide as a colorless oil.

### (2E,4E)-6-(Pentyloxy)hexa-2,4-dien-1-amine

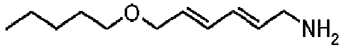

Under nitrogen atmosphere, lithium aluminum hydride (1.0 M in Et₂O, 1.308 ml) was diluted with dry diethyl ether (20 ml) and cooled in an ice bath. A solution of (2E,4E)-1-azido-6-(pentyloxy)hexa-2,4-diene (2.05 g) in dry diethyl ether (5 ml) was added dropwise. The mixture was stirred at 0°C for 1 h, the ice bath was removed and the mixture was stirred for 1 h while warming to room temperature. Water was added carefully until gas evolution ceased and the orange mixture turned pink, before it turns light yellow. The mixture was filtered, dried over sodium sulfate, filtered again and concentrated *in vacuo* to yield the crude amine as a yellow liquid. The amine was used as such.

### 3-Butoxypropanal

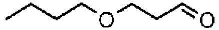

A solution of 3-butoxypropanenitrite (31.2 g) in dry tetrahydrofuran (500 ml) was cooled under nitrogen atmosphere to -20°C. DiBALH (1M in hexanes, 417 ml) was added dropwise and the mixture was stirred for 2 h at -20°C, after which it was warmed to room temperature and stirred overnight. The reaction mixture was cooled to 0°C (dry ice/acetone) and kept at this temperature while the mixture was hydrolyzed by adding 1M HCl dropwise (400 ml). Concentrated HCl (∼100 ml) and water (∼200 ml) were added and the aqueous layer was extracted twice with Et₂O (∼400ml each). The combined organic layer was washed with brine, dried over Na₂SO₄ and concentrated *in vacuo* at 300 mbar, 40°C to yield the aldehyde as an orange liquid. The product was used as such for the next step.

### Ethyl (2E)-5-butoxypent-2-enoate

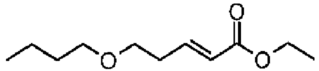

Under nitrogen atmosphere, sodium hydride (60% wt., 10.58 g) was suspended in dry tetrahydrofuran (700 ml) and cooled to 0°C. Triethyl phosphonoacetate (52.5 ml) was added dropwise and the mixture was stirred for 30 min at 0°C. The mixture was cooled to -78°C and 3-butoxypropanal (28.7 g) in dry tetrahydrofuran (50 ml) was added dropwise. Stirring was continued at -78°C for 1.5 h. The mixture was allowed to warm to room temperature and hydrolyzed with water. Water was added and the aqueous layer was extracted with EtOAc. The combined organic layer was washed with brine, dried over Na₂SO₄ and concentrated *in vacuo* to yield an orange oil, which was purified by column chromatography (0-10% EtOAc in heptane) to yield the desired product as a colorless oil.

### (2E)-5-Butoxypent-2-en-1-ol

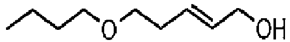

A solution of (*E*)-ethyl 5-butoxypent-2-enoate (19.41 g) in dry toluene (200 ml) was cooled to -78°C. DiBALH (1M in hexanes, 213 ml) was added dropwise and the mixture was stirred at this temperature for 1.5 h. The dry ice/acetone bath was removed and the mixture was stirred for another 1 h. The mixture was hydrolyzed with 1M HCl (aq) (cooling in ice/water bath). Additional water and concentrated HCl (aq) were added (pH = 1). The layers were separated and the aqueous layer was extracted twice with EtOAc. The combined organic layer was washed with brine, dried over Na₂SO₄ and concentrated *in vacuo* to yield the alcohol as a colorless oil. The product was used as such in the next step.

### (2E)-5-Butoxypent-2-enal

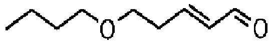

(*E*)-5-butoxypent-2-en-1-ol (11.99 g) was dissolved in dichloromethane (150 ml) and the mixture was cooled in an ice bath. Dess-Martin periodinane (33.6 g) was added portionwise and the mixture was stirred at room temperature for 15 min (white suspension). The reaction mixture was hydrolyzed with saturated NaHCO₃ (aq) and filtered. The aqueous layer was extracted twice with CH₂Cl₂. The combined organic layer was washed with brine, dried over Na₂SO₄ and concentrated *in vacuo.* The concentrate was dissolved in a minimum amount of CH₂Cl₂ (solution) and Et₂O was added (suspension). The solids were filtered off and the filtrate was concentrated *in vacuo* to yield the aldehyde. The product was used as such in the next step.

### Ethyl (2E,4E)-7-butoxyhepta-2,4-dienoate

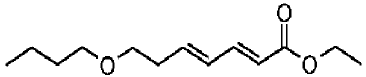

Sodium hydride (60% wt., 3.24 g) was suspended in dry tetrahydrofuran (100 ml) and cooled to 0°C. Triethyl phosphonoacetate (16.05 ml) was added dropwise. The reaction mixture was stirred at 0°C for 30 min and cooled to -78°C after which (*E*)-5-butoxypent-2-enal (11.7 g) in dry tetrahydrofuran (50 ml) was added dropwise. The reaction mixture was stirred at this temperature for 1 h and allowed to warm to room temperature. The reaction was hydrolyzed with water/THF, poured into water and the product was extracted twice with EtOAc. The combined organic layer was washed with brine, dried over Na₂SO₄, and concentrated *in vacuo.* The crude material was purified via column chromatography (0-5% EtOAc in heptane) to yield the ester.

### (2E,4E)-7-Butoxyhepta-2,4-dien-1-ol

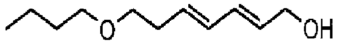

A solution of (*2E*,*4E*)-ethyl 7-butoxyhepta-2,4-dienoate (12.9 g) in dry toluene (180 ml) was cooled to -78°C. DiBALH (1M in hexanes, 117 ml) was added dropwise and the mixture was stirred at this temperature for 1.5 h. The dry ice/acetone bath was removed and the mixture was stirred for another 1 h. The mixture was hydrolyzed with 1M HCl (aq) (cooling in ice/water bath). Additional water and concentrated HCl (aq) were added (pH = 1). The layers were separated and the aqueous layer was extracted twice with EtOAc. The combined organic layer was washed with brine, dried over Na₂SO₄ and concentrated *in vacuo.* The alcohol was used as such in the next step.

### (2E,4E)-1-Azido-7-butoxyhepta-2,4-diene

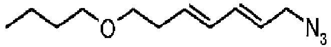

(*2E,4E*)-7-butoxyhepta-2,4-dien-1-ol (8.11 g, 44.0 mmol) was dissolved in dry toluene (100 ml), cooled in an ice bath and diphenyl phosphorazidate (9.54 ml) was added dropwise. DBU (7.89 ml) was added dropwise. The mixture was stirred at room temperature overnight. The solvent was evaporated and the residue was partitioned between diethyl ether and 1M NaOH. The organic layer was separated and washed with 0.5M HCl, water and brine, dried over Na₂SO₄ and concentrated *in vacuo.* The crude product was filtered over a plug of silica (0-5% EtOAc in heptane) to yield the azide as a colorless oil.

### (2E,4E)-7-Butoxyhepta-2,4-dien-1-amine

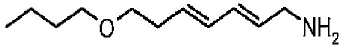

Under nitrogen atmosphere, 4M LiAlH₄ in diethyl ether (5.73 ml) was diluted with dry diethyl ether (400 ml) and cooled in an ice bath. A solution of (*2E,4E*)-1-azido-7-butoxyhepta-2,4-diene (7.99 g) in dry diethyl ether (50 ml) was added dropwise. The mixture was stirred at 0°C for 1 h, the ice bath was removed and the mixture was stirred for 1 h while warming to room temperature. Water (∼6 ml) was added carefully until gas evolution ceased. The mixture was dried over Na₂SO₄ and concentrated *in vacuo* to yield the product as a yellow liquid and was used as such in the next step.

### tert-Butyl [(2E,4E)-7-butoxyhepta-2,4-dien-1-yl]carbamate

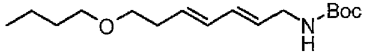

To a cooled (ice bath) solution of (*2E,4E*)-7-butoxyhepta-2,4-dien-1-amine (6.84 g) and triethylamine (5.34 ml) in dichloromethane (400 ml) was added di-tert-butyl dicarbonate (7.74 g). The resulting mixture was stirred at room temperature overnight. Water was added, the phases separated and the aqueous layer was washed with CH₂Cl₂. The combined organic layer was dried over Na₂SO₄, and concentrated *in vacuo.* The crude product was purified via column chromatography (0-5% EtOAc in heptane) to yield the product as a colorless oil.

### tert-Butyl methyl[(2E,4E)-7-butoxyhepta-2,4-dien-1-yl]carbamate

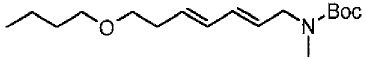

Sodium hydride (60% wt., 0.141 g) was washed with heptane. Dry N,N-dimethylformamide (5 ml) was added. The mixture was cooled to 0°C with an ice bath and tert-butyl (*2E*,*4E*)-7-butoxyhepta-2,4-dienylcarbamate (1 g) in dry N,N-dimethylformamide (5 ml) was added dropwise. The reaction mixture was stirred for 30 min after which methyl iodide (0.331 ml) was added. After complete addition the reaction mixture was warmed to room temperature and stirred for 2 h. The reaction mixture was poured into water, extracted twice with EtOAc. The EtOAc-layers were combined and washed with brine, dried over Na₂SO₄ and concentrated *in vacuo.* The crude product was purified via column chromatography (0-30% EtOAc in heptane) to yield the product as colorless oil.

### Synthesis of Example 3:

### Example 3:

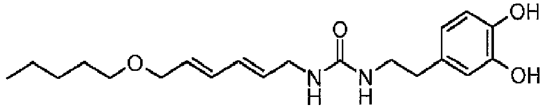

To a solution of (*2E,4E*)-6-(pentyloxy)hexa-2,4-dien-1-amine (100 mg) in dichloromethane (1 ml) was added 1,1'-carbonyldiimidazole (80 mg). The mixture was stirred at room temperature for 3 h. 3,4-Dihydroxyphenethylamine hydrochloride (93 mg) and triethylamine (68 *µ*l) were mixed in dichloromethane (1 ml) and added to the reaction mixture solution. The reaction mixture was heated at 40°C for 4 days. The reaction mixture was concentrated *in vacuo* and purified via flash column chromatography and reversed phase column chromatography to yield the desired product.

### Synthesis of Example 5:

### Intermediate 5a):

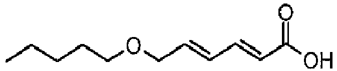

(2E,4E)-Ethyl 6-(pentyloxy)hexa-2,4-dienoate (200 mg) was dissolved in methanol (5 ml). 2M Sodium hydroxide (aq) (0.574 ml) was added and the resulting suspension was heated to 60°C for 3 h. The reaction mixture was cooled to room temperature and acidified with 1M HCl (aq). The product was extracted twice with EtOAc. The combined organic layer was washed with brine, dried over Na₂SO₄ and concentrated *in vacuo* to yield the product as yellow oil.

### Example 5:

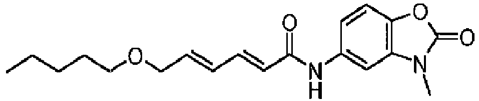

To a suspension of 5-amino-3-methyl-1,3-benzoxazol-2(3H)-one (158 mg) and (*2E,4E*)-6-(pentyloxy)hexa-2,4-dienoic acid (173 mg) in dichloromethane (3 ml) and dry N,N-dimethylformamide (2 ml) was added EDCI.HCl (184 mg) followed by 1-hydroxy-7-azabenzotriazole (11.88 mg) and the reaction mixture was stirred at room temperature overnight. The mixture was concentrated *in vacuo* and purified via flash column chromatography to yield the product as an off white solid.

### Synthesis of Example 9:

### Intermediate 9a):

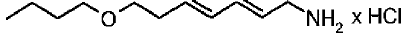

tert-Butyl (*2E,4E*)-7-butoxyhepta-2,4-dienylcarbamate (3 g) was dissolved in hydrochloric acid in dioxane (4.0M, 26.5 ml) and the mixture was stirred at room temperature for 1 h. The mixture was concentrated and stripped with Et₂O twice. Et₂O was added and the solid was filtered off to yield the amine hydrochlorid as a white solid.

### Example 9:

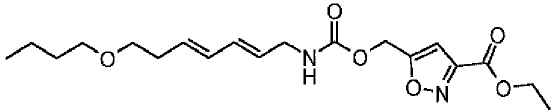

Ethyl 5-(hydroxymethyl)isoxazole-3-carboxylate (prepared according to WO2010/142801, p. 147, 0.093 g) was dissolved in dichloromethane (5 ml) and 1,1'-carbonyldimidazole (0.089 g) was added. The reaction mixture was stirred for 2 h at room temperature before (*2E,4E*)-7-butoxyhepta-2,4-dien-1-amine hydrochloride (0.120 g) and triethylamine (0.076 ml) were added. The reaction mixture was warmed to 40°C and stirred overnight after which it was cooled to room temperature and washed with water. The organic layer was collected and evaporated *in vacuo.* The crude product was purified via flash column chromatography to yield the product as a white solid.

### Synthesis of Example 16:

### Intermediate 16a):

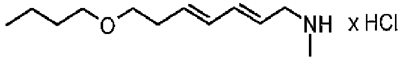

tert-Butyl (*2E,4E*)-7-butoxyhepta-2,4-dienyl(methyl)carbamate (770 mg) was dissolved in dry diethyl ether (5 ml) and 4M HCl in dioxane (6.47 ml) was added. The reaction mixture was stirred for 3 h at room temperature. The solvents were evaporated *in vacuo* and co-evaporated twice with Et₂O. A sticky white solid was obtained which was used immediately for the next step.

### Intermediate 16b):

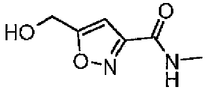

Ethyl 5-(hydroxymethyl)isoxazole-3-carboxylate (0.270 g) was dissolved in absolute ethanol (4.5 ml). Methylamine (2M in THF, 3.16 ml) was added. The vial was sealed and stirred at 70°C overnight. The reaction mixture was cooled to room temperature and the solvent was evaporated *in vacuo.* The crude product was purified via flash column chromatography (1-4% methanol in dichloromethane) affording the product as a white solid.

***Example 16:***

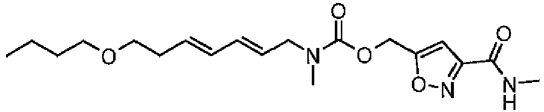

5-(Hydroxymethyl)-N-methylisoxazole-3-carboxamide (66.8 mg) was dissolved in dry tetrahydrofuran (2 ml). To this was added p-nitrophenyl chloroformate (86 mg) and triethylamine (0.060 ml). The reaction mixture was stirred at room temperature for 3 h. (*2E,4E*)-7-Butoxy-N-methylhepta-2,4-dien-1-amine hydrochloride (100 mg) in dry tetrahydrofuran (1 ml) was added, followed by triethylamine (0.060 ml). The reaction mixture was heated to 60°C overnight after which it was cooled to room temperature and poured into water. DCM was added, the mixture stirred vigorously, and filtered over a phase separator. The solvent was evaporated *in vacuo* and the crude product was purified via flash column chromatography (30%-100% EtOAc in heptane) to yield the product as a colorless oil.

Examples 1-2, 4, 6-8, 10-15 and 17-18 as indicated in Table 1 above have been prepared in an analogous manner as Examples 3, 5, 9 and 16 using the respective starting compounds.

### BIOLOGICAL ASSAYS

### A. Radioligand Displacement Assays on CB₁ and CB₂ Receptors

For the CB₁ receptor, binding experiments were performed in the presence of 0.5 nM of the radioligand [³H]CP55,940 (168 Ci/mmol) (Perkin Elmer, Waltham, MA, US) at 30°C in siliconized glass vials together with 8.0 µg of membrane recombinantly over-expressing CB₁ (No. RBHCB1M, Perkin Elmer, Waltham, MA, US), which was resuspended in 0.5 ml (final volume) assay-buffer (50 mM TRIS-HCl, 2.5 mM EDTA, 5 mM MgCl₂, 0.5 mg/ml fatty acid free BSA, pH 7.4). Test compounds were present at varying concentrations and the nonspecific binding of the radioligand was determined in the presence of 10 µM WIN55,212-2 (Tocris Cookson Ltd., Bristol, UK). After 2 h incubation, the suspension was rapidly filtered through 0.1 % polyethylenimine pre-soaked UniFilter®-96 GFB (Perkin Elmer, Boston MA, US) washed twelve times with 167 µl ice-cold assay-buffer. Radioactivity on dried and sealed filter plates was measured with a Perkin Elmer 1450 Microbeta TRILUX liquid scintillation counter in 40 µL MicroScint 20 scintillation cocktail (Perkin Elmer, Waltham, MA, US). Data collected from three independent experiments performed in triplicates were normalized between 100% and 0% specific binding for [³H]CP55,940. These data were graphically linearized by projecting Hill-plots, which allowed the calculation of IC₅₀ values. Derived from the dissociation constant (K*_{D}*) of [³H]CP55,940 and the concentration-dependent displacement (IC₅₀ value), inhibition constants (*Kᵢ*) of competitor compounds were calculated using the Cheng-Prusoff equation [*Kᵢ* = IC₅₀/(1 + L/K_{D})].

For CB₂-receptor binding studies 3.8 µg of membrane recombinantly over-expressing CB₂ (No. RBXCB2M, Perkin Elmer, Waltham, MA, US) were resuspended in 0.5 ml assay buffer (see above) together with 0.5 nM of the radioligand [³H]CP55,940. The CB₂-binding assay was conducted in the same manner as for CB₁.

### B. Critical micelle concentration (CMC) measurements

Dye micellization was carried out with a modified version of the method described by Eliyahu et al., Novel dextran-spermine conjugates as transfecting agents: comparing water-soluble and micellar polymers. Gene Ther. 2005 Mar;12(6):494-503. Compounds (from 2 mM stock solutions) were mixed at increasing concentrations with 0.1 nM fluorescein (free acid, 99%, Fluka, Switzerland) at room temperature in Nanopure distilled water. Experiments were carried out on 96-well microtiter plates (excitation at 485 nm, emission at 535 nm). Since the emission of fluorescein is pH-dependent, the pH of the mixture was kept constant at 6.9. The CMC range was determined as the concentration range where the first statistically significant increase in fluorescence was detected. At an appropriate resolution of the concentration range this increase was not gradual but sudden.

### C. Cellular uptake of [³H]-AEA

Human lymphoma U937 cells were grown in RPMI 1640 medium (Invitrogen, Basel, Switzerland) supplemented with 10% fetal bovine serum, 1 g/ml fungizone (amphotericin B), 100 units/ml penicillin, 100 g/ml streptomycin, and 2 mM L-glutamine (all from Invitrogen) and selected for the cellular AEA uptake assay. 2.5 x 10⁶ cells were collected and suspended in 250 µL of RPMI 1640 medium (Invitrogen, Basel, Switzerland) at 37°C using glass silanized vials. Cells were pre-incubated 15 min at 37°C in presence of test compounds in a concentration range of 10⁻¹¹ - 10⁻⁵ M or in presence of the same amount (5 µl) of vehicle control (DMSO). Then a mixture of 0.5 nM [³H]-AEA (60 Ci/mmol) (American Radiolabeled Chemicals, Inc., San Louis, MO, US) and 99.5 nM of nonradioactive AEA (Tocris Cookson Ltd., Bristol, UK) resulting in a final concentration of 100 nM AEA was added and samples were incubated at 37°C for another 15 minutes. The reaction was stopped by rapid filtration over UniFilter®-96 GF/C (Perkin Elmer, Boston MA, US) pre-soaked with PBS 0.25 % BSA (w/v). Cells were washed three times with ice-cold PBS buffer containing 1% fatty acid free BSA (137 mM NaCl, 2.7 mM KCl, 10 mM Na₂HPO₄, 1.76 mM KH₂PO₄, 1% BSA fatty acid free, pH 7.4), dried and 40 µL MicroScint 20 scintillation cocktail (Perkin Elmer, Waltham, MA, US) was added. Radioactivity was measured using a Perkin Elmer 1450 Microbeta TRILUX liquid scintillation counter. Results were expressed as % of AEA uptake versus cells treated with solvent and the EC₅₀ and IC₅₀ values were calculated from the sigmoidal curves, which were generated using the GraphPad Prism 5.0 (GraphPad Software, San Diego, CA, US).

**Table 2: Inhibition of Arachidonoylethanolamide Transport (AEAT) (Determination as Described in Biological Assays C)**

| Example | AEAT inhibition at 250 nM | AEAT inhibition at 1 µM | AEAT |
|---|---|---|---|
| | % of control | % of control | EC₅₀ (nM) |
| 1 | | | 147 |
| 2 | 95 | 29 | |
| 3 | | | 6.0 |
| 4 | -2 | -16 | |
| 5 | 82 | 36 | |
| 6 | 44 | 70 | |
| 7 | 86 | 28 | |
| 8 | 72 | 36 | |
| 9 | | | 3.0 |
| 10 | 48 | 56 | |
| 11 | 56 | 32 | |
| 12 | | | 6.0 |
| 13 | | | 0.573 |
| 14 | | | 286 |
| 15 | | | 70 |
| 16 | | | 7.0 |
| 17 | | | 81 |
| 18 | | | 9.0 |

### D. Fatty acid amide hydrolase (FAAH) inhibition

FAAH activity was assessed using pig brain or U937 homogenate according to the method described by Omeir et. al. Arachidonoyl ethanolamide-[1,2-14C] as a substrate for anandamide amidase. Life sciences 56, 1999-2005 (1995) and adapted by Fowler et al. Selective inhibition of anandamide cellular uptake versus enzymatic hydrolysis--a difficult issue_ to handle. European journal of pharmacology 492, 1-11 (2004).

Briefly, 10 µl of vehicle or positive control URB597 (Cayman Chemical, Ann Arbor, MI, US) resulting a final concentration of 100 nM or of the inhibitor at the adequate concentrations were pre-incubated at 37°C for 15 min at 450 rpm with 490 µl of diluted pig brain (200 µg per sample) or U937 homogenate (0.63 mg protein per sample corr. 10⁶ U937 cells) in FAAH activity buffer (10 mM Tris-HCl and 1 mM EDTA, pH 8.0, 0.1 % w/v fatty acid free BSA (Sigma, San Louis, MO, US)). A mixture of 0.5 nM [³H]AEA (60 Ci/mmol) and 99.5 nM of nonradioactive AEA resulting in a total concentration of 100 nM AEA was added and incubated for 15 min. Successively, 1 ml of a mixture 1:1 (v/v) of methanol:chloroform was added to each sample and, after vigorous vortexing, aqueous and organic phases were separated by centrifugation at 10'000 rpm for 10 min at 4°C. The radioactivity associated to the [³H]-ethanolamine (produced by FAAH-catalysed breakdown of [³H]-AEA) was measured upon addition of 3 ml of Ultima Gold scintillation liquid (Perkin Elmer, Waltham, MA, US) to the aqueous (upper) phase, using a PACKARD TRI-CARB 2100TR Liquid Scintillation Analyzer. Results were expressed as % of FAAH activity vs. vehicle treated homogenate. Collected data was normalized by subtraction of unspecific signal at total inhibition of FAAH by URB597. Single values of FAAH inhibition or IC₅₀ values from generated sigmoidal curves were calculated using GraphPad Prism 5.0 (GraphPad Software, San Diego, CA, US).

### E. Monoacylglycerol lipase (MAGL) inhibition

MAGL activity was evaluated using the Monoacylglycerol Lipase Inhibitor Screening Assay Kit (Cayman Chemicals, MI, US) and the experimental procedure was carried out according to the kit protocol. Briefly, 10 µl of test compounds at different concentrations (range 0.1-20 µM) were added to 150 µl of assay buffer, 10 µl of human recombinant MAGL and 10 µl of arachidonoyl-1-thio-glycerol (150 µM in the assay). The plate was incubated at room temperature for 5 min, afterward 10 µl of DTNB (5, 5'-dithiobis-(2-nitrobenzoic acid), Ellman's reagent) was added to each well. The plate was shaken for 10 seconds and the absorbance measured at 415 nm using Genius Pro spectrofluorimeter (Tecan, Grodig, Austria). Initial 100% enzymatic activty was calulated using solvent instead of test compounds, whilst background was measured without the human recombinant enzyme. The blank was subtracted from each value and results were expressed as % of initial enzymatic activity.

### F. In vivo model for pruritus associated with the oxazolone model of a delayed type hypersensitivity reaction

Scratching activity in mice is measured after topical application of the test compound. Ear thickness is measured and histology parameters are determined (see e.g. Elliott G.R. An automated method for registering and quantifying scratching activity in mice: use for drug evaluation. J. Pharmacol. Toxicol. Methods. 2000;44:453-459 and Gijbels M.J. Therapeutic interventions in mice with chronic proliferative dermatitis (cpdm/cpdm). Exp. Dermatol. 2000;9:351-358).

### Examples of a Pharmaceutical Composition

### Composition Example 9:

| **Cream** | |
|---|---|
| Compound Example 9 | 1.00 |
| Cetostearyl alcohol | 7.00 |
| Macrogol-6-cetostearyl ether | 1.50 |
| Macrogol-25-cetoostearyl ether | 1.50 |
| Liquid paraffin | 12.00 |
| Propylene glycol | 8.00 |
| Methylparaben | 0.15 |
| Ethylparaben | 0.08 |
| Butylhydroxytoluene | 0.04 |
| Disodium edetate | 0.05 |
| Water | 68.68 |

### Composition Example 13:

| **Gel** | |
|---|---|
| Compound Example 13 | 0.50 |
| Ethanol | 15.00 |
| Polyoxyl 40 Hydrogenated Castor Oil | 1.00 |
| Butylhydroxytoluene | 0.04 |
| Disodium edetate | 0.05 |
| Carbomer | 0.50 |
| Triethanolamine | 0.70 |
| Water | 82.21 |

### Industrial Applicability - Utility

The above results clearly show the improved technical effects provided by the *E,E*-diene compounds of the present invention. It has surprisingly been found out by the present inventors that these compounds are suitable as active ingredients in pharmaceutical and cosmetic compositions, preferably dermatologic agents. Moreover, the compounds have shown to be effective in treating and/or preventing several diseases and conditions including, but not limited to inflammation, irritation, itching, pruritus, pain, oedema, and/or pro-allergic or allergic conditions, in particular when they are topically applied to the skin or mucosa in the form of pharmaceutical or cosmetic compositions containing a suitable carrier, preferably an apolar carrier. In particular, inflammation induced by cellular stress signals can effectively be attenuated by these compounds. Moreover, the compounds of the invention are effective in the treatment/prevention of hair growth conditions (e.g. alopecia, effluvium), sebaceous gland disorders (e.g. acne, seborrhea), benign and malignant skin tumors, hyperproliferative skin diseases (e.g. psoriasis), excessive hair growth (e.g. hirsutism), different forms of dermatitis, dry skin conditions and/or systemic sclerosis (e.g. scleroderma). The compounds of formulae (1) to (7) are modulators of the endocannabinoid system and as such are useful in the treatment, control or prevention of the above mentioned diseases, disorders or conditions being responsive to one or more constituents of the endocannabinoid system including, but not limited to CB₁ and CB₂, TRPV1, GPR55, FAAH, monoacylglyceol lipase (MAGL) or AEAT. The pharmaceutical (dermatological) compositions and preparations containing the compounds of the present invention are highly active, even if the amount of active compound is reduced as compared to prior art compounds, and show less undesirable side-effects.

While the invention has been described and illustrated in reference to certain preferred embodiments thereof, those skilled in the art will appreciate that various changes, modifications and substitutions can be made therein without departing from the spirit and the scope of the invention. For example, effective dosages, other than the preferred doses as set forth above, may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound of formula (1) 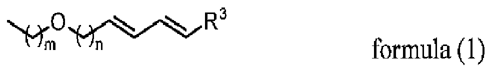 and the enantiomers, diastereomers, tautomers, solvates and pharmaceutically acceptable salts thereof,
wherein
R³ is selected from the group consisting of
-CH₂N(R⁴)C(O)R¹
-CH₂N(R⁴)C(O)NR¹R²
-CH₂N(R⁴)C(O)OR¹
-C(O)NR¹R²;
wherein
n is 1, 2, 3, 4, or 5;
m is 0, 1, 2, 3, or 4;
m + n is 1, 2, 3, 4, 5, 6 or 7;
wherein
R¹ and R² are independently selected from hydrogen, C₁₋₆ alkyl, C₃₋₇ cycloalkyl, C₆₋₁₀ aryl, 5-to 10-membered heteroaryl, (C₆₋₁₀ aryl)-C₁₋₆ alkyl, (5- to 10-membered heteroaryl)-C₁₋₆ alkyl, (C₆₋₁₀ aryl fused with a 4 to 8-membered saturated or unsaturated heterocyclyl containing 1 to 3 heteroatoms independently selected from N, O, S), (C₆₋₁₀ aryl fused with a 4 to 8-membered saturated or unsaturated heterocyclyl containing 1 to 3 heteroatoms independently selected from N, O, S)-C₁₋₆alkyl, C₁₋₆alkyl-C(O)OC₁₋₆alkyl, C₁₋₆alkyl-C(O)NH(C₁₋₆alkyl), C₁₋₆alkyl-C(O)N(C₁₋₆alkyl)₂, C₁₋₆alkyl-CN;
R⁴ is hydrogen or C₁₋₆-alkyl;
and wherein R¹, R², and R⁴, independently of each other, may be substituted by one, two or three substituents R⁵;
wherein R⁵ is selected from the group consisting of
halogen,
CN,
OH,
Oxo,
C₁₋₆-alkyl, optionally substituted with one or more substituents selected from halogen,
OH, OC₁₋₆alkyl, OC₃₋₇cycloalkyl,
OC₁₋₆-alkyl, optionally substituted with one or more substituents selected from halogen, OH, OC₁₋₆alkyl, OC₃₋₇cycloalkyl,
C₆₋₁₀-aryl
5- to 10-membered heteroaryl,
5- to 11-membered heterocyclyl, optionally substituted with one or more substituents selected from halogen, OH, oxo, OC₁₋₆alkyl, OC₃₋₇cycloalkyl C(O)NH₂,
C(O)OH,
C(O)OC₁₋₆alkyl, optionally substituted with one or more substituents selected from halogen, OH, OC₁₋₆alkyl, OC₃₋₇cycloalkyl,
C(O)OC₃₋₇cycloalkyl, optionally substituted with one or more substituents selected from halogen, OH, OC₁₋₆alkyl, OC₃₋₇cycloalkyl,
C(O)NH-C₁₋₆alkyl, optionally substituted with one or more substituents selected from halogen, OH, OC₁₋₆alkyl, OC₃-₇cycloalkyl,
C(O)NH-C₃₋₇cycloalkyl, optionally substituted with one or more substituents selected from halogen, OH, OC₁₋₆alkyl, OC₃₋₇cycloalkyl,
C(O)N-(C₁₋₆alkyl)₂, optionally substituted with one or more substituents selected from halogen, OH, OC₁₋₆alkyl, OC₃₋₇cycloalkyl,
C(O)N-(C₁₋₆alkyl)(C₃₋₇cycloalkyl), optionally substituted with one or more substituents selected from halogen, OH, OC₁₋₆alkyl, OC₃₋₇cycloalkyl, SO₂-C₁₋₆alkyl, optionally substituted with one or more substituents selected from halogen, OH, OC₁₋₆alkyl, OC₃₋₇cycloalkyl,
NH₂,
NH-C₁₋₆alkyl, optionally substituted with one or more substituents selected from halogen, OH, OC₁₋₆alkyl, OC₃₋₇cycloalkyl,
N-(C₁₋₆alkyl)₂, optionally substituted with one or more substituents selected from halogen, OH, OC₁₋₆alkyl, OC₃₋₇cycloalkyl,
NH-SO₂-CH₃,
NH-C(O)OC₁₋₆alkyl, optionally substituted with one or more substituents selected from halogen, OH, OC₁₋₆alkyl, OC₃₋₇cycloalkyl,
NH-C(O)OC₃₋₇cycloalkyl, optionally substituted with one or more substituents selected from halogen, OH, OC₁₋₆alkyl, OC₃₋₇cycloalkyl,
N(C₁₋₆alkyl)-C(O)OC₁₋₆alkyl, optionally substituted with one or more substituents selected from halogen, OH, OC₁₋₆alkyl, OC₃₋₇cycloalkyl,
N(C₁₋₆alkyl)-C(O)OC₃₋₇cycloalkyl, optionally substituted with one or more substituents selected from halogen, OH, OC₁₋₆alkyl, OC₃₋₇cycloalkyl,
NH-C(O)C₁₋₆alkyl, optionally substituted with one or more substituents selected from halogen, OH, OC₁₋₆alkyl, OC₃₋₇cycloalkyl,
NH-C(O)C₃-₇cycloalkyl, optionally substituted with one or more substituents selected from halogen, OH, OC₁₋₆alkyl, OC₃₋₇cycloalkyl,
N(C₁₋₆alkyl)-C(O)C₁₋₆alkyl, optionally substituted with one or more substituents selected from halogen, OH, OC₁₋₆alkyl, OC₃₋₇cycloalkyl,
N(C₁₋₆alkyl)-C(O)C₃₋₇cycloakyl, optionally substituted with one or more substituents selected from halogen, OH, OC₁₋₆alkyl, OC₃₋₇cycloalkyl.

2. The compound according to Claim 1, wherein in formula. (1) n is 1 or 2, preferably 2;
m is 3 or 4, preferably 3, and
R³ is selected from the group consisting of
-CH₂N(R⁴)C(O)OR¹
-CH₂N(R⁴)C(O)NR¹R².

3. The compound according to any one of claims 1 and 2, wherein,
in the NR¹R² moieties, one of R¹ and R² is hydrogen and the other is selected from (C₆₋₁₀ aryl)-C₁₋₆ alkyl, (5- to 10-membered heteroaryl)-C₁₋₆ alkyl, (C₆₋₁₀ aryl fused with a 4 to 8-membered saturated or unsaturated heterocyclyl containing 1 to 3 heteroatoms independently selected from N, O, S), (C₆₋₁₀ aryl fused with a 4 to 8-membered saturated or unsaturated heterocyclyl containing 1 to 3 heteroatoms independently selected from N, O, S)-C₁₋₆alkyl; and in the C(O)R¹ and C(O)OR¹ moieties, R¹ is selected from C₆₋₁₀ aryl, 5- to 10-membered heteroaryl, (C₆₋₁₀ aryl)-C₁₋₆ alkyl, (5- to 10-membered heteroaryl)-C₁₋₆ alkyl, (C₆₋₁₀ aryl fused with a 4 to 8-membered saturated or unsaturated heterocyclyl containing 1 to 3 heteroatoms independently selected from N, O, S), (C₆₋₁₀ aryl fused with a 4 to 8-membered saturated or unsaturated heterocyclyl containing 1 to 3 heteroatoms independently selected from N, O, S)-C₁₋₆alkyl, C₁₋₆alkyl-C(O)OC₁₋₆alkyl, C₁₋₆alkyl-C(O)NH(C₁₋₆alkyl), C₁₋₆alkyl-C(O)N(C₁₋₆ alkyl)₂, C₁₋₆alkyl-CN.

4. The compound according to any one of claims 1 to 3,
wherein R³ is -CH₂N(R⁴)C(O)OR¹, R¹ is selected from C₆₋₁₀ aryl, (C₆₋₁₀ aryl)-C₁₋₆ alkyl, C₁₋₆ alkyl-CN, C₁₋₆alkyl-C(O)NH(C₁₋₆alkyl), C₁₋₆alkyl-C(O)N(C₁₋₆alkyl)₂, and (5- to 10-membered heteroaryl)-C₁₋₆ alkyl; and R⁴ is either hydrogen or methyl.

5. The compound according to any one of claims 1 to 4,
wherein R¹ and R², independently of each other, are substituted by one or two substituents R⁵; wherein R⁵ is selected from the group consisting of
OH,
OXO,
C₁₋₆-alkyl,
OC₁₋₆-alkyl,
C(O)NH₂,
C(O)OH,
C(O)OC₁₋₆alkyl,
C(O)NH-C₁₋₆alkyl,
C(O)N-(C₁₋₆alkyl)₂.

6. The compound according to claim 5,
wherein R⁵ is selected from the group consisting of
OH,
OXO,
C₁₋₃-alkyl,
OC₁₋₃-alkyl,
C(O)NH₂,
C(O)OH,
C(O)OC₁₋₃alkyl,
C(O)NH-C₁₋₃alkyl,
C(O)N-(C₁₋₃alkyl)₂.

7. The compound according to claim 6,
wherein R⁵ is selected from the group consisting of
OH,
OXO,
methyl,
Omethyl,
C(O)NH₂,
C(O)OH,
C(O)Omethyl,
C(O)NH-methyl,
C(O)N-(methyl)₂.

8. The compound according to any one of claims 1 to 7 for use as a medicament, preferably as a dermatologic agent.

9. The compound according to any one of claims 1 to 8 for use in treating and/or preventing inflammation, irritation, itching, pruritus, pain, oedema and/or pro-allergic or allergic conditions, preferably on the skin and/or mucosa of a mammal, and/or alopecia, effluvium, sebaceous gland disorders, benign and malignant skin tumors, hyperproliferative skin diseases, excessive hair growth, dermatitis, dry skin conditions and/or systemic sclerosis.

10. The compound according to any one of claims 1 to 9 which is applied topically on the skin or mucosa of a mammal.

11. Non-therapeutic use of the compound as defined in any one of claims 1-7 as a cosmetic, preferably for the cosmetic treatment of the skin and/or mucosa of a mammal.

12. Non-therapeutic use according to claim 11 for reducing inflammation, irritation, itching, pruritus, pain, oedema and/or pro-allergic or allergic conditions, and alopecia, effluvium, sebaceous gland disorders, hyperproliferative skin diseases, excessive hair growth, dermatitis, dry skin conditions and/or systemic sclerosis.

13. Composition comprising the compound as defined in any one of claims 1-10 and a pharmaceutically and/or cosmetically acceptable carrier, preferably an apolar carrier, wherein the composition preferably is a liniment, skin cream, gel, or lotion.
